(19) Europäisches Patentamt
European Patent Office
Office européen des brevets

(11) EP 2 963 121 A1

(12) EUROPEAN PATENT APPLICATION

(43) Date of publication:
06.01.2016 Bulletin 2016/01

(51) Int Cl.:
*C12P 13/00* (2006.01) *C12N 9/06* (2006.01)

(21) Application number: 14175632.0

(22) Date of filing: 03.07.2014

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: BASF SE
67056 Ludwigshafen (DE)

(72) Inventors:
- Baldenius, Kai-Uwe Dr., 68159 Mannheim (DE)
- Breuer, Michael Dr., 64285 Darmstadt (DE)
- Ditrich, Klaus Prof.Dr., 67161 Gönnheim (DE)
- Navickas, Vaidotas Dr., 68199 Mannheim (DE)
- Mutti, Francesco Dr., 1079 CV Amsterdam (NL)
- Knaus, Tanja Dr., 1079 CV Amsterdam (NL)
- Turner, Nicholas Prof.Dr., M20 3DH Manchester (GB)

(74) Representative: Reitstötter Kinzebach
Patentanwälte
Sternwartstrasse 4
81679 München (DE)

# (54) Redox self-sufficient biocatalytic amination of alcohols

(57) The present invention relates to a novel biocatalytic method for the production of primary and secondary amines, comprising coupled enzymatic oxidation and reduction processes simultaneously regenerating the required cofactor; making use of two enzymes - i.e. an alcohol dehydrogenase and an amine dehydrogenase - operating simultaneously in a one pot process (i.e. biocatalytic cascade). Furthermore, the overall process is redox neutral, since the hydride generated from the first oxidative step is internally recycled in the second reductive step. The invention also relates to recombinant expression systems and microorganisms procuring the required enzyme activities; and bioreactors for performing such methods.

EP 2 963 121 A1

SEQUENCE LISTING

<110>  BASF SE

<120>  Redox self-sufficient biocatalytic amination of alcohols

<130>  M/54453

<160>  10

<170>  PatentIn version 3.5

<210>  1
<211>  380
<212>  PRT
<213>  Bacillus badius

<400>  1

```
Met Ser Leu Val Glu Lys Thr Ser Ile Ile Lys Asp Phe Thr Leu Phe
1               5                   10                  15

Glu Lys Met Ser Glu His Glu Gln Val Val Phe Cys Asn Asp Pro Ala
            20                  25                  30

Thr Gly Leu Arg Ala Ile Ile Ala Ile His Asp Thr Thr Leu Gly Pro
        35                  40                  45

Ala Leu Gly Gly Cys Arg Met Gln Pro Tyr Asn Ser Val Glu Glu Ala
    50                  55                  60

Leu Glu Asp Ala Leu Arg Leu Ser Lys Gly Met Thr Tyr Lys Cys Ala
65                  70                  75                  80

Ala Ser Asp Val Asp Phe Gly Gly Gly Lys Ala Val Ile Ile Gly Asp
                85                  90                  95

Pro Gln Lys Asp Lys Ser Pro Glu Leu Phe Arg Ala Phe Gly Gln Phe
            100                 105                 110

Val Asp Ser Leu Gly Gly Arg Phe Tyr Thr Gly Thr Asp Met Gly Thr
        115                 120                 125

Asn Met Glu Asp Phe Ile His Ala Met Lys Glu Thr Asn Cys Ile Val
    130                 135                 140

Gly Val Pro Glu Ala Tyr Gly Gly Gly Gly Asp Ser Ser Ile Pro Thr
145                 150                 155                 160

Ala Met Gly Val Leu Tyr Gly Ile Lys Ala Thr Asn Lys Met Leu Phe
                165                 170                 175
```

Gly Lys Asp Asp Leu Gly Gly Val Thr Tyr Ala Ile Gln Gly Leu Gly
180 185 190

Lys Val Gly Tyr Lys Val Ala Glu Gly Leu Leu Glu Glu Gly Ala His
195 200 205

Leu Phe Val Thr Asp Ile Asn Glu Gln Thr Leu Glu Ala Ile Gln Glu
210 215 220

Lys Ala Lys Thr Thr Ser Gly Ser Val Thr Val Val Ala Ser Asp Glu
225 230 235 240

Ile Tyr Ser Gln Glu Ala Asp Val Phe Val Pro Cys Ala Phe Gly Gly
245 250 255

Val Val Asn Asp Glu Thr Met Lys Gln Phe Lys Val Lys Ala Ile Ala
260 265 270

Gly Ser Ala Asn Asn Gln Leu Leu Thr Glu Asp His Gly Arg His Leu
275 280 285

Ala Asp Lys Gly Ile Leu Tyr Ala Pro Asp Tyr Ile Val Asn Ser Gly
290 295 300

Gly Leu Ile Gln Val Ala Asp Glu Leu Tyr Glu Val Asn Lys Glu Arg
305 310 315 320

Val Leu Ala Lys Thr Lys His Ile Tyr Asp Ala Ile Leu Glu Val Tyr
325 330 335

Gln Gln Ala Glu Leu Asp Gln Ile Thr Thr Met Glu Ala Ala Asn Arg
340 345 350

Met Cys Glu Gln Arg Met Ala Ala Arg Gly Arg Arg Asn Ser Phe Phe
355 360 365

Thr Ser Ser Val Lys Pro Lys Trp Asp Ile Arg Asn
370 375 380

<210> 2
<211> 400
<212> PRT
<213> Artificial Sequence

<220>
<223> PhDH Mutant

<400> 2

Met Gly Ser Ser His His His His His His Ser Ser Gly Leu Val Pro

1 5 10 15

Arg Gly Ser His Met Ser Leu Val Glu Lys Thr Ser Ile Ile Lys Asp
20 25 30

Phe Thr Leu Phe Glu Lys Met Ser Glu His Glu Gln Val Val Phe Cys
35 40 45

Asn Asp Pro Ala Thr Gly Leu Arg Ala Ile Ile Ala Ile His Asp Thr
50 55 60

Thr Leu Gly Pro Ala Leu Gly Gly Cys Arg Met Gln Pro Tyr Asn Ser
65 70 75 80

Val Glu Glu Ala Leu Glu Asp Ala Leu Arg Leu Ser Lys Gly Met Thr
85 90 95

Tyr Ser Cys Ala Ala Ser Asp Val Asp Phe Gly Gly Gly Lys Ala Val
100 105 110

Ile Ile Gly Asp Pro Gln Lys Asp Lys Ser Pro Glu Leu Phe Arg Ala
115 120 125

Phe Gly Gln Phe Val Asp Ser Leu Gly Gly Arg Phe Tyr Thr Gly Thr
130 135 140

Asp Met Gly Thr Asn Met Glu Asp Phe Ile His Ala Met Lys Glu Thr
145 150 155 160

Asn Cys Ile Val Gly Val Pro Glu Ala Tyr Gly Gly Gly Gly Asp Ser
165 170 175

Ser Ile Pro Thr Ala Met Gly Val Leu Tyr Gly Ile Lys Ala Thr Asn
180 185 190

Lys Met Leu Phe Gly Lys Asp Asp Leu Gly Gly Val Thr Tyr Ala Ile
195 200 205

Gln Gly Leu Gly Lys Val Gly Tyr Lys Val Ala Glu Gly Leu Leu Glu
210 215 220

Glu Gly Ala His Leu Phe Val Thr Asp Ile Asn Glu Gln Thr Leu Glu
225 230 235 240

Ala Ile Gln Glu Lys Ala Lys Thr Thr Ser Gly Ser Val Thr Val Val
245 250 255

Ala Ser Asp Glu Ile Tyr Ser Gln Glu Ala Asp Val Phe Val Pro Cys
260 265 270

Ala Phe Gly Gly Val Val Asn Asp Glu Thr Met Lys Gln Phe Lys Val
275 280 285

Lys Ala Ile Ala Gly Ser Ala Asn Leu Gln Leu Leu Thr Glu Asp His
290 295 300

Gly Arg His Leu Ala Asp Lys Gly Ile Leu Tyr Ala Pro Asp Tyr Ile
305 310 315 320

Val Asn Ser Gly Gly Leu Ile Gln Val Ala Asp Glu Leu Tyr Glu Val
325 330 335

Asn Lys Glu Arg Val Leu Ala Lys Thr Lys His Ile Tyr Asp Ala Ile
340 345 350

Leu Glu Val Tyr Gln Gln Ala Glu Leu Asp Gln Ile Thr Thr Met Glu
355 360 365

Ala Ala Asn Arg Met Cys Glu Gln Arg Met Ala Ala Arg Gly Arg Arg
370 375 380

Asn Ser Phe Phe Thr Ser Ser Val Lys Pro Lys Trp Asp Ile Arg Asn
385 390 395 400

<210> 3
<211> 429
<212> PRT
<213> Bacillus stearothermophilus

<400> 3

Met Glu Leu Phe Lys Tyr Met Glu Thr Tyr Asp Tyr Glu Gln Val Leu
1 5 10 15

Phe Cys Gln Asp Lys Glu Ser Gly Leu Lys Ala Ile Ile Ala Ile His
20 25 30

Asp Thr Thr Leu Gly Pro Ala Leu Gly Gly Thr Arg Met Trp Met Tyr
35 40 45

Asn Ser Glu Glu Glu Ala Leu Glu Asp Ala Leu Arg Leu Ala Arg Gly
50 55 60

Met Thr Tyr Lys Asn Ala Ala Ala Gly Leu Asn Leu Gly Gly Gly Lys
65 70 75 80

```
Thr Val Ile Ile Gly Asp Pro Arg Lys Asp Lys Asn Glu Ala Met Phe
                85                  90                  95

Arg Ala Phe Gly Arg Phe Ile Gln Gly Leu Asn Gly Arg Tyr Ile Thr
            100                 105                 110

Ala Glu Asp Val Gly Thr Thr Val Ala Asp Met Asp Ile Ile Tyr Gln
        115                 120                 125

Glu Thr Asp Tyr Val Thr Gly Ile Ser Pro Glu Phe Gly Ser Ser Gly
    130                 135                 140

Asn Pro Ser Pro Ala Thr Ala Tyr Gly Val Tyr Arg Gly Met Lys Ala
145                 150                 155                 160

Ala Ala Lys Glu Ala Phe Gly Ser Asp Ser Leu Glu Gly Lys Val Val
                165                 170                 175

Ala Val Gln Gly Val Gly Asn Val Ala Tyr His Leu Cys Arg His Leu
            180                 185                 190

His Glu Glu Gly Ala Lys Leu Ile Val Thr Asp Ile Asn Lys Glu Val
        195                 200                 205

Val Ala Arg Ala Val Glu Glu Phe Gly Ala Lys Ala Val Asp Pro Asn
    210                 215                 220

Asp Ile Tyr Gly Val Glu Cys Asp Ile Phe Ala Pro Cys Ala Leu Gly
225                 230                 235                 240

Gly Ile Ile Asn Asp Gln Thr Ile Pro Gln Leu Lys Ala Lys Val Ile
                245                 250                 255

Ala Gly Ser Ala Asp Asn Gln Leu Lys Glu Pro Arg His Gly Asp Ile
            260                 265                 270

Ile His Glu Met Gly Ile Val Tyr Ala Pro Asp Tyr Val Ile Asn Ala
        275                 280                 285

Gly Gly Val Ile Asn Val Ala Asp Glu Leu Tyr Gly Tyr Asn Arg Glu
    290                 295                 300

Arg Ala Met Lys Lys Ile Glu Gln Ile Tyr Asp Asn Ile Glu Lys Val
305                 310                 315                 320

Phe Ala Ile Ala Lys Arg Asp Asn Ile Pro Thr Tyr Val Ala Ala Asp
                325                 330                 335
```

```
Arg Met Ala Glu Glu Arg Ile Glu Thr Met Arg Lys Ala Ala Ser Gln
            340                 345                 350

Phe Leu Gln Asn Gly His His Ile Leu Ser Arg Arg Pro Arg Pro Leu
        355                 360                 365

Thr Ala Ala Arg Ala Gly Leu Arg Arg Ala Asp Asp Gly Gly Thr Thr
    370                 375                 380

Thr Met Gln Glu Gln Lys Phe Arg Ile Leu Thr Ile Asn Pro Gly Ser
385                 390                 395                 400

Thr Ser Thr Lys Ile Gly Val Phe Glu Asn Glu Arg Ala Ile Ala Ser
                405                 410                 415

Lys Lys Arg Ser Ala Thr Arg Ala Gly Ala Ser Ala Ile
            420                 425
```

<210> 4
<211> 429
<212> PRT
<213> Artificial Sequence

<220>
<223> L-DH Mutant

<400> 4

```
Met Glu Leu Phe Lys Tyr Met Glu Thr Tyr Asp Tyr Glu Gln Val Leu
1               5                   10                  15

Phe Cys Gln Asp Lys Glu Ser Gly Leu Lys Ala Ile Ile Ala Ile His
            20                  25                  30

Asp Thr Thr Leu Gly Pro Ala Leu Gly Gly Thr Arg Met Trp Met Tyr
        35                  40                  45

Asn Ser Glu Glu Glu Ala Leu Glu Asp Ala Leu Arg Leu Ala Arg Gly
    50                  55                  60

Met Thr Tyr Ser Asn Ala Ala Ala Gly Leu Asn Leu Gly Gly Gly Lys
65                  70                  75                  80

Thr Val Ile Ile Gly Asp Pro Arg Lys Asp Lys Asn Glu Ala Met Phe
                85                  90                  95

Arg Ala Phe Gly Arg Phe Ile Gln Gly Leu Asn Gly Arg Tyr Ile Thr
            100                 105                 110
```

```
Ala Val Asp Val Gly Thr Thr Val Ala Asp Met Asp Ile Ile Tyr Gln
        115                 120                 125

Glu Thr Asp Tyr Val Thr Gly Ile Ser Pro Glu Phe Gly Ser Ser Gly
    130                 135                 140

Asn Pro Ser Pro Ala Thr Ala Tyr Gly Val Tyr Arg Gly Met Lys Ala
145                 150                 155                 160

Ala Ala Lys Glu Ala Phe Gly Ser Asp Ser Leu Glu Gly Lys Val Val
                165                 170                 175

Ala Val Gln Gly Val Gly Asn Val Ala Tyr His Leu Cys Arg His Leu
            180                 185                 190

His Glu Glu Gly Ala Lys Leu Ile Val Thr Asp Ile Asn Lys Glu Val
        195                 200                 205

Val Ala Arg Ala Val Glu Glu Phe Gly Ala Lys Ala Val Asp Pro Asn
    210                 215                 220

Asp Ile Tyr Gly Val Glu Cys Asp Ile Phe Ala Pro Cys Ala Leu Gly
225                 230                 235                 240

Gly Ile Ile Asn Asp Gln Thr Ile Pro Gln Leu Lys Ala Lys Val Ile
                245                 250                 255

Ala Gly Ser Ala Asp Leu Gln Leu Lys Glu Pro Arg His Gly Asp Ile
            260                 265                 270

Ile His Glu Met Gly Ile Val Tyr Ala Pro Asp Tyr Val Ile Asn Ala
        275                 280                 285

Gly Gly Cys Ile Asn Val Ala Asp Glu Leu Tyr Gly Tyr Asn Arg Glu
    290                 295                 300

Arg Ala Met Lys Lys Ile Glu Gln Ile Tyr Asp Asn Ile Glu Lys Val
305                 310                 315                 320

Phe Ala Ile Ala Lys Arg Asp Asn Ile Pro Thr Tyr Val Ala Ala Asp
                325                 330                 335

Arg Met Ala Glu Glu Arg Ile Glu Thr Met Arg Lys Ala Ala Ser Gln
            340                 345                 350

Phe Leu Gln Asn Gly His His Ile Leu Ser Arg Arg Pro Arg Pro Leu
        355                 360                 365
```

Thr Ala Ala Arg Ala Gly Leu Arg Arg Ala Asp Asp Gly Gly Thr Thr
370 375 380

Thr Met Gln Glu Gln Lys Phe Arg Ile Leu Thr Ile Asn Pro Gly Ser
385 390 395 400

Thr Ser Thr Lys Ile Gly Val Phe Glu Asn Glu Arg Ala Ile Ala Ser
405 410 415

Lys Lys Arg Ser Ala Thr Arg Ala Gly Ala Ser Ala Ile
420 425

<210> 5
<211> 249
<212> PRT
<213> Arothron maculatus

<400> 5

Met Thr Gln Arg Leu Lys Asp Lys Leu Ala Val Ile Thr Gly Gly Ala
1 5 10 15

Asn Gly Ile Gly Arg Ala Ile Ala Glu Arg Phe Ala Val Glu Gly Ala
20 25 30

Asp Ile Ala Ile Ala Asp Leu Val Pro Ala Pro Glu Ala Glu Ala Ala
35 40 45

Ile Arg Asn Leu Gly Arg Arg Val Leu Thr Val Lys Cys Asp Val Ser
50 55 60

Gln Pro Gly Asp Val Glu Ala Phe Gly Lys Gln Val Ile Ser Thr Phe
65 70 75 80

Gly Arg Cys Asp Ile Leu Val Asn Asn Ala Gly Ile Tyr Pro Leu Ile
85 90 95

Pro Phe Asp Glu Leu Thr Phe Glu Gln Trp Lys Lys Thr Phe Glu Ile
100 105 110

Asn Val Asp Ser Gly Phe Leu Met Ala Lys Ala Phe Val Pro Gly Met
115 120 125

Lys Arg Asn Gly Trp Gly Arg Ile Ile Asn Leu Thr Ser Thr Thr Tyr
130 135 140

Trp Leu Lys Ile Glu Ala Tyr Thr His Tyr Ile Ser Thr Lys Ala Ala
145 150 155 160

```
Asn Ile Gly Phe Thr Arg Ala Leu Ala Ser Asp Leu Gly Lys Asp Gly
                165                 170                 175

Ile Thr Val Asn Ala Ile Ala Pro Ser Leu Val Arg Thr Ala Thr Thr
            180                 185                 190

Glu Ala Ser Ala Leu Ser Ala Met Phe Asp Val Leu Pro Asn Met Leu
        195                 200                 205

Gln Ala Ile Pro Arg Leu Gln Val Pro Leu Asp Leu Thr Gly Ala Ala
    210                 215                 220

Ala Phe Leu Ala Ser Asp Asp Ala Ser Phe Ile Thr Gly Gln Thr Leu
225                 230                 235                 240

Ala Val Asp Gly Gly Met Val Arg His
                245


<210>  6
<211>  251
<212>  PRT
<213>  Lactobacillus brevis

<400>  6

Ser Asn Arg Leu Asp Gly Lys Val Ala Ile Ile Thr Gly Gly Thr Leu
1               5                   10                  15

Gly Ile Gly Leu Ala Ile Ala Thr Lys Phe Val Glu Glu Gly Ala Lys
            20                  25                  30

Val Met Ile Thr Asp Arg His Ser Asp Val Gly Glu Lys Ala Ala Lys
        35                  40                  45

Ser Val Gly Thr Pro Asp Gln Ile Gln Phe Phe Gln His Asp Ser Ser
    50                  55                  60

Asp Glu Asp Gly Trp Thr Lys Leu Phe Asp Ala Thr Glu Lys Ala Phe
65                  70                  75                  80

Gly Pro Val Ser Thr Leu Val Asn Asn Ala Gly Ile Ala Val Asn Lys
                85                  90                  95

Ser Val Glu Glu Thr Thr Thr Ala Glu Trp Arg Lys Leu Leu Ala Val
            100                 105                 110

Asn Leu Asp Gly Val Phe Phe Gly Thr Arg Leu Gly Ile Gln Arg Met
        115                 120                 125
```

```
Lys Asn Lys Gly Leu Gly Ala Ser Ile Ile Asn Met Ser Ser Ile Glu
    130                 135                 140

Gly Phe Val Gly Asp Pro Ser Leu Gly Ala Tyr Asn Ala Ser Lys Gly
145                 150                 155                 160

Ala Val Arg Ile Met Ser Lys Ser Ala Ala Leu Asp Cys Ala Leu Lys
                165                 170                 175

Asp Tyr Asp Val Arg Val Asn Thr Val His Pro Gly Tyr Ile Lys Thr
            180                 185                 190

Pro Leu Val Asp Asp Leu Pro Gly Ala Glu Glu Ala Met Ser Gln Arg
        195                 200                 205

Thr Lys Thr Pro Met Gly His Ile Gly Glu Pro Asn Asp Ile Ala Tyr
    210                 215                 220

Ile Cys Val Tyr Leu Ala Ser Asn Glu Ser Lys Phe Ala Thr Gly Ser
225                 230                 235                 240

Glu Phe Val Val Asp Gly Gly Tyr Thr Ala Gln
                245                 250


<210>  7
<211>  260
<212>  PRT
<213>  Bacillus subtilis

<400>  7

Met Tyr Pro Asp Leu Lys Gly Lys Val Val Ala Ile Thr Gly Ala Ala
1               5                   10                  15

Ser Gly Leu Gly Lys Ala Met Ala Ile Arg Phe Gly Lys Glu Gln Ala
            20                  25                  30

Lys Val Val Ile Asn Tyr Tyr Ser Asn Lys Gln Asp Pro Asn Glu Val
        35                  40                  45

Lys Glu Glu Val Ile Lys Ala Gly Gly Glu Ala Val Val Val Gln Gly
    50                  55                  60

Asp Val Thr Lys Glu Glu Asp Val Lys Asn Ile Val Gln Thr Ala Ile
65                  70                  75                  80

Lys Glu Phe Gly Thr Leu Asp Ile Met Ile Asn Asn Ala Gly Leu Glu
                85                  90                  95
```

```
Asn Pro Val Pro Ser His Glu Met Pro Leu Lys Asp Trp Asp Lys Val
            100                 105                 110

Ile Gly Thr Asn Leu Thr Gly Ala Phe Leu Gly Ser Arg Glu Ala Ile
        115                 120                 125

Lys Tyr Phe Val Glu Asn Asp Ile Lys Gly Asn Val Ile Asn Met Ser
    130                 135                 140

Ser Val His Ala Phe Pro Trp Pro Leu Phe Val His Tyr Ala Ala Ser
145                 150                 155                 160

Lys Gly Gly Ile Lys Leu Met Thr Glu Thr Leu Ala Leu Glu Tyr Ala
                165                 170                 175

Pro Lys Gly Ile Arg Val Asn Asn Ile Gly Pro Gly Ala Ile Asn Thr
            180                 185                 190

Pro Ile Asn Ala Glu Lys Phe Ala Asp Pro Lys Gln Lys Ala Asp Val
        195                 200                 205

Glu Ser Met Ile Pro Met Gly Tyr Ile Gly Glu Pro Glu Glu Ile Ala
    210                 215                 220

Ala Val Ala Ala Trp Leu Ala Ser Lys Glu Ala Ser Tyr Val Thr Gly
225                 230                 235                 240

Ile Thr Leu Phe Ala Asp Gly Gly Met Thr Gln Tyr Pro Ser Phe Gln
                245                 250                 255

Ala Gly Arg Gly
            260


<210>  8
<211>  395
<212>  PRT
<213>  Equus caballus

<400>  8

Met Gly Ser Ser His His His His His His Ser Ser Gly Leu Val Pro
1               5                   10                  15

Arg Gly Ser His Met Ser Thr Ala Gly Lys Val Ile Lys Cys Lys Ala
            20                  25                  30

Ala Val Leu Trp Glu Glu Lys Lys Pro Phe Ser Ile Glu Glu Val Glu
        35                  40                  45
```

```
Val Ala Pro Pro Lys Ala His Glu Val Arg Ile Lys Met Val Ala Thr
    50                  55                  60

Gly Ile Cys Arg Ser Asp Asp His Val Val Ser Gly Thr Leu Val Thr
65                  70                  75                  80

Pro Leu Pro Val Ile Ala Gly His Glu Ala Ala Gly Ile Val Glu Ser
                85                  90                  95

Ile Gly Glu Gly Val Thr Thr Val Arg Pro Gly Asp Lys Val Ile Pro
            100                 105                 110

Leu Phe Thr Pro Gln Cys Gly Lys Cys Arg Val Cys Lys His Pro Glu
        115                 120                 125

Gly Asn Phe Cys Leu Lys Asn Asp Leu Ser Met Pro Arg Gly Thr Met
    130                 135                 140

Gln Asp Gly Thr Ser Arg Phe Thr Cys Arg Gly Lys Pro Ile His His
145                 150                 155                 160

Phe Leu Gly Thr Ser Thr Phe Ser Gln Tyr Thr Val Val Asp Glu Ile
                165                 170                 175

Ser Val Ala Lys Ile Asp Ala Ala Ser Pro Leu Glu Lys Val Cys Leu
            180                 185                 190

Ile Gly Cys Gly Phe Ser Thr Gly Tyr Gly Ser Ala Val Lys Val Ala
        195                 200                 205

Lys Val Thr Gln Gly Ser Thr Cys Ala Val Phe Gly Leu Gly Gly Val
    210                 215                 220

Gly Leu Ser Val Ile Met Gly Cys Lys Ala Ala Gly Ala Ala Arg Ile
225                 230                 235                 240

Ile Gly Val Asp Ile Asn Lys Asp Lys Phe Ala Lys Ala Lys Glu Val
                245                 250                 255

Gly Ala Thr Glu Cys Val Asn Pro Gln Asp Tyr Lys Lys Pro Ile Gln
            260                 265                 270

Glu Val Leu Thr Glu Met Ser Asn Gly Gly Val Asp Phe Ser Phe Glu
        275                 280                 285

Val Ile Gly Arg Leu Asp Thr Met Val Thr Ala Leu Ser Cys Cys Gln
    290                 295                 300
```

```
Glu Ala Tyr Gly Val Ser Val Ile Val Gly Val Pro Pro Asp Ser Gln
305                 310                 315                 320

Asn Leu Ser Met Asn Pro Met Leu Leu Leu Ser Gly Arg Thr Trp Lys
                325                 330                 335

Gly Ala Ile Phe Gly Gly Phe Lys Ser Lys Asp Ser Val Pro Lys Leu
            340                 345                 350

Val Ala Asp Phe Met Ala Lys Lys Phe Ala Leu Asp Pro Leu Ile Thr
        355                 360                 365

His Val Leu Pro Phe Glu Lys Ile Asn Glu Gly Phe Asp Leu Leu Arg
    370                 375                 380

Ser Gly Glu Ser Ile Arg Thr Ile Leu Thr Phe
385                 390                 395


<210>  9
<211>  345
<212>  PRT
<213>  Rhodococcus ruber

<400>  9

Met Lys Ala Val Gln Tyr Thr Glu Ile Gly Ser Glu Pro Val Val Val
1               5                   10                  15

Asp Ile Pro Thr Pro Thr Pro Gly Pro Gly Glu Ile Leu Leu Lys Val
            20                  25                  30

Thr Ala Ala Gly Leu Cys His Ser Asp Ile Phe Val Met Asp Met Pro
        35                  40                  45

Ala Ala Gln Tyr Ala Tyr Gly Leu Pro Leu Thr Leu Gly His Glu Gly
    50                  55                  60

Val Gly Thr Val Ala Glu Leu Gly Glu Gly Val Thr Gly Phe Gly Val
65                  70                  75                  80

Gly Asp Ala Val Ala Val Tyr Gly Pro Trp Gly Cys Gly Ala Cys His
                85                  90                  95

Ala Cys Ala Arg Gly Arg Glu Asn Tyr Cys Thr Arg Ala Ala Asp Leu
            100                 105                 110

Gly Ile Thr Pro Pro Gly Leu Gly Ser Pro Gly Ser Met Ala Glu Tyr
        115                 120                 125
```

```
Met Ile Val Asp Ser Ala Arg His Leu Val Pro Ile Gly Asp Leu Asp
    130                 135                 140

Pro Val Ala Ala Ala Pro Leu Thr Asp Ala Gly Leu Thr Pro Tyr His
145                 150                 155                 160

Ala Ile Ser Arg Val Leu Pro Leu Leu Gly Pro Gly Ser Thr Ala Val
                165                 170                 175

Val Ile Gly Val Gly Gly Leu Gly His Val Gly Ile Gln Ile Leu Arg
            180                 185                 190

Ala Val Ser Ala Ala Arg Val Ile Ala Val Asp Leu Asp Asp Asp Arg
        195                 200                 205

Leu Ala Leu Ala Arg Glu Val Gly Ala Asp Ala Ala Val Lys Ser Gly
    210                 215                 220

Ala Gly Ala Ala Asp Ala Ile Arg Glu Leu Thr Gly Gly Gln Gly Ala
225                 230                 235                 240

Thr Ala Val Phe Asp Phe Val Gly Ala Gln Ser Thr Ile Asp Thr Ala
                245                 250                 255

Gln Gln Val Val Ala Val Asp Gly His Ile Ser Val Val Gly Ile His
            260                 265                 270

Ala Gly Ala His Ala Lys Val Gly Phe Phe Met Ile Pro Phe Gly Ala
        275                 280                 285

Ser Val Val Thr Pro Tyr Trp Gly Thr Arg Ser Glu Leu Met Glu Val
    290                 295                 300

Val Ala Leu Ala Arg Ala Gly Arg Leu Asp Ile His Thr Glu Thr Phe
305                 310                 315                 320

Thr Leu Asp Glu Gly Pro Ala Ala Tyr Arg Arg Leu Arg Glu Gly Ser
                325                 330                 335

Ile Arg Gly Arg Gly Val Val Val Pro
            340                 345

<210>  10
<211>  337
<212>  PRT
<213>  Geobacillus stearothermophilus
```

<400> 10

```
Met Lys Ala Ala Val Val Glu Gln Phe Lys Lys Pro Leu Gln Val Lys
1               5                   10                  15

Glu Val Glu Lys Pro Lys Ile Ser Tyr Gly Glu Val Leu Val Arg Ile
            20                  25                  30

Lys Ala Cys Gly Val Cys His Thr Asp Leu His Ala Ala His Gly Asp
        35                  40                  45

Trp Pro Val Lys Pro Lys Leu Pro Leu Ile Pro Gly His Glu Gly Val
    50                  55                  60

Gly Val Ile Glu Glu Val Gly Pro Gly Val Thr His Leu Lys Val Gly
65                  70                  75                  80

Asp Arg Val Gly Ile Pro Trp Leu Tyr Ser Ala Cys Gly His Cys Asp
                85                  90                  95

Tyr Cys Leu Ser Gly Gln Glu Thr Leu Cys Glu Arg Gln Gln Asn Ala
            100                 105                 110

Gly Tyr Ser Val Asp Gly Gly Tyr Ala Glu Tyr Cys Arg Ala Ala Ala
        115                 120                 125

Asp Tyr Val Val Lys Ile Pro Asp Asn Leu Ser Phe Glu Glu Ala Ala
    130                 135                 140

Pro Ile Phe Cys Ala Gly Val Thr Thr Tyr Lys Ala Leu Lys Val Thr
145                 150                 155                 160

Gly Ala Lys Pro Gly Glu Trp Val Ala Ile Tyr Gly Ile Gly Gly Leu
                165                 170                 175

Gly His Val Ala Val Gln Tyr Ala Lys Ala Met Gly Leu Asn Val Val
            180                 185                 190

Ala Val Asp Leu Gly Asp Glu Lys Leu Glu Leu Ala Lys Gln Leu Gly
        195                 200                 205

Ala Asp Leu Val Val Asn Pro Lys His Asp Asp Ala Ala Gln Trp Ile
    210                 215                 220

Lys Glu Lys Val Gly Gly Val His Ala Thr Val Val Thr Ala Val Ser
225                 230                 235                 240

Lys Ala Ala Phe Glu Ser Ala Tyr Lys Ser Ile Arg Arg Gly Gly Ala
```

```
                245                 250                 255

Cys Val Leu Val Gly Leu Pro Pro Glu Glu Ile Pro Ile Pro Ile Phe
            260                 265                 270

Asp Thr Val Leu Asn Gly Val Lys Ile Ile Gly Ser Ile Val Gly Thr
        275                 280                 285

Arg Lys Asp Leu Gln Glu Ala Leu Gln Phe Ala Ala Glu Gly Lys Val
    290                 295                 300

Lys Thr Ile Val Glu Val Gln Pro Leu Glu Asn Ile Asn Asp Val Phe
305                 310                 315                 320

Asp Arg Met Leu Lys Gly Gln Ile Asn Gly Arg Val Val Leu Lys Val
                325                 330                 335

Asp
```

## Description

The present invention relates to a novel biocatalytic method for the production of amines, comprising coupled enzymatic oxidation and reduction processes simultaneously regenerating the required cofactor; making use of two enzymes - i.e. an alcohol dehydrogenase and an amine dehydrogenase - operating simultaneously in a one pot process (i.e. biocatalytic cascade). Furthermore, the overall process is redox neutral, since the hydride generated from the first oxidative step is internally recycled in the second reductive step. Thus external reducing equivalents are not required; such a system is defined as redox self-sufficient. The invention also relates to recombinant expression systems and microorganisms procuring the required enzyme activities; and bioreactors for performing such methods.

## Background of the invention

Hydroxyl functionalities are ubiquitously found in renewable resources and bio-based feedstocks (e.g. alcohols, carbohydrates), whereas amine moieties are scarcer in nature. However, amines are widely used in the production of pharmaceuticals, fine chemicals, agrochemicals, polymers, dyestuffs, pigments, emulsifiers and plasticizing agents. Among the different types of amines, primary amines are in particular useful intermediates for industrial applications. Therefore the functional group interconversion of an alcohol (primary or secondary) into the corresponding primary amine is still of great interest in organic synthesis. In fact, the production of amines on the laboratory as well as industrial scale mainly relies on the reductive amination of the corresponding carbonyl compounds. In contrast the direct amination of an alcohol in a redox neutral fashion allows maximizing the atom efficiency of the process since external redox equivalents are not required. Heterogeneously catalyzed amination of simple alcohols such as methanol and ethanol are performed on a multi-thousands-ton-scale. (K. Weissermel, H.-J. Arpe, Industrial Organic Chemistry, 4th ed.,Wiley-VCH, Weinheim, 2003, p. 51). Due to the limited activity of the heterogeneous catalysts, relatively harsh conditions (> 200 °C) are required for alcohol amination. Moreover, more structurally diverse alcohols are either converted with extremely low chemoselectivity or not converted at all.

The redox neutral direct synthesis of amines starting from alcohols is possible using the $[Ru(CO)ClH(PPh_3)_3]$ Xantphos catalyst in a homogeneous system using *tert*-amyl alcohol as solvent. (S. Imm, S. Bähn, M. Zhang, L. Neubert, H. Neumann, F. Klasovsky, J. Pfeffer, T. Haas, and M. Beller, Angew. Chem. Int. Ed. 2011, 50, 7599 - 7603). The Ru catalyst abstracts the hydride from the alcohol in the first oxidative step to give the carbonyl compound as intermediate and the Ru-H species. In presence of ammonia, the carbonyl compound is in equilibrium with the related imine. Hence, the Ru-H form of the catalyst transfers the hydride back to the imine intermediate, finally affording the amine product. The main disadvantages of the chemocatalytic process are:

1) the low chemoselectivity (*i.e.* the primary amine generated after the first catalytic cycle can compete with the ammonia in the following cycles, leading to a mixture of primary, secondary and even tertiary amines;
2) the lack of stereoselectivity,
3) the extremely low substrate concentration (1 mM);
4) the high catalyst loading (3 mol%);
5) the relatively harsh reaction conditions (150 °C, ammonia as gas under pressure);
6) the need for argon atmosphere.

Although most reported work in this area involves non-chiral substrates, a stereoselective hydrogen-borrowing amination of alcohols has been recently published (Y. Zhang, C.-S. Lim, D. S. B. Sim, H.-J. Pan, Y. Zhao, Angew. Chem. Int. Ed. 2014, 126, 1423-1427). However, also this method is plagued by several economic as well as environmental limitations:

1) this method requires a high loading (5 mol%) of an iridium catalyst coordinated to a complex and expensive organic chiral ligand;
2) this method requires a high loading (10 mol%) of an expensive binaphtalen chiral phosphoric acid as co-catalyst, otherwise conversion is not observed at all;
3) this method requires more complex primary amines than ammonia (i.e. *para*-anisidine and few other phenylamine derivatives) as the nitrogen source.; as a consequence primary amines from alcohols cannot be directly obtained, only secondary phenyl-substituted amines can be obtained; it is interesting to note that, among the different types of amines, primary amines are in particular useful intermediates for industrial applications;
4) this method requires 1.5 equivalents of the alcohol for the amination with *para*-anisidine; hence at least 33% of the alcohol is wasted even if a theoretical quantitative yield is obtained;
5) enantiomeric excess is moderate with few exceptions. However, enantiomeric excess was never superior to 97%;

Recently, a biocatalytic hydrogen-borrowing amination of primary alcohols has been proposed using three enzymes: an alcohol dehydrogenase (ADH), a ω-transaminase (ω-TA) and an alanine dehydrogenase (Ala-DH) (Scheme 1) (J. H. Sattler, M. Fuchs, K.Tauber, F. G. Mutti, K. Faber, J. Pfeffer, T. Haas, W. Kroutil, Angew. Chem. Int. Ed. 2012, 51, 9156-9159.)

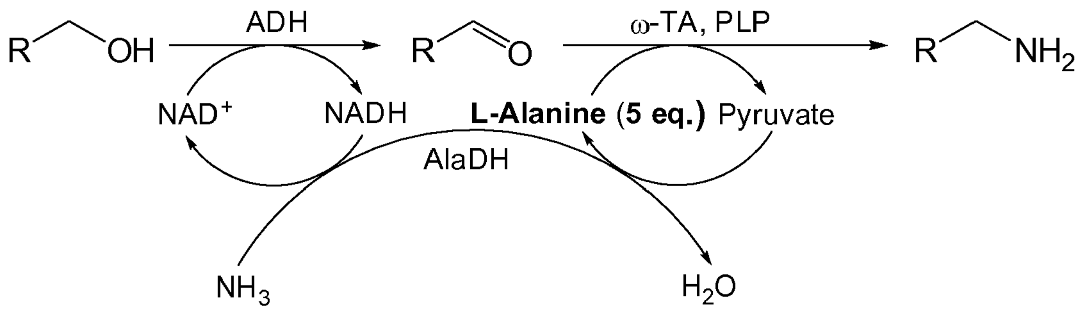


**Scheme 1. Three enzymes cascade for the amination of primary alcohols.**

In the first step, the ADH oxidises the alcohol to the aldehyde generating the reduced form of the NAD cofactor. In the second step, the ω-TA formally transfers the amino group from the amino donor (L-alanine) to the aldehyde intermediate. Pyruvate is produced as side product besides the desired amine. Finally, the Ala-DH allows for shifting the equilibrium via recycling the pyruvate back to L-alanine employing ammonia and the reducing equivalents (*i.e.* NADH) generated in the first step.
Nevertheless one must notice that:

1) The cycle is not truly catalytic. At least 5 equivalents of L-alanine are required to observe the formation of the amine product.
2) This biocatalytic network afforded >99% amine conversion only starting from primary alcohol.
3) The same concept applied to secondary alcohols led to poorer conversions (54% or lower). Moreover, only enantiopure S-configured alcohols were partially converted into S-configured amines (only amination with retention of configuration).
4) ω-Transaminases are very often plagued by substrate (e.g. ketone / aldehyde, alanine) and product (e.g. amine, pyruvate) inhibition (a) J.-S. Shin, B.-G. Kim, Biosci. Biotechnol. Biochem. 2001, 65, 1782 - 1788; b) J.-S. Shin, B.-G. Kim, Biotechnol. Bioeng. 1999, 65, 206 - 211; c) J.-S. Shin, B.-G. Kim, Biotechnol. Bioeng. 2002, 77, 832 - 837).
5) The use of a three enzyme system complicates the set-up of the reaction and complicates the possibility for multiple gene cloning into the same host organism. Furthermore L-alanine has to be supplied in the system.
6) When using (*R*)-selective ω-TAs, the more expensive D-alanine must be used. Furthermore, an alanine dehydrogenase capable of recycling the pyruvate back to D-alanine is not known. Only L-alanine would be produced. Hence, in the case of (*R*)-selective ω-TAs, the system works via a removal of pyruvate rather than a recycling of the pyruvate. This point also conflicts with the definition of a "catalytic process".

It is evident, that there is a need for further improved biocatalytic methods for preparing amines.

## Summary of the invention

Multi-step chemical reactions in one pot in sequential as well as in concurrent fashion avoid the need for isolation of intermediates and purification steps. This approach leads to economic as well as environmental benefits since time-consuming intermediate work-ups are not required and the use of organic solvents for extraction / purification and energy for evaporation / mass transfer is minimized. Therefore cascade reactions generally possess elevated atom efficiency and lower environmental impact factors. The major challenge is to perform cascade reactions wherein an oxidative and a reductive step are running simultaneously. Even more challenging is carrying out a simultaneous interconnected redox neutral cascade wherein the electrons liberated in the first oxidative step are quantitatively consumed in the subsequent reductive step. This concept is the basis for the present invention concerning the interconversion of alcohols (primary or secondary) into amines.

Surprisingly the above mentioned problem could be solved by providing biocatalytic method as defined in the attached claims.

In particular, the problem was solved by providing a new enzymatic catalytic method for the redox self-sufficient (*i.e.* hydrogen-borrowing) amination of alcohols by combining only two biocatalysts, namely an alcohol dehydrogenase

(ADH) with an amine dehydrogenase (AmDH),

For example by applying said enzymes in vitro as purified enzymes conversions exceeding 94% and perfect chemo- and stereoselectivity are achieved (Scheme 2):

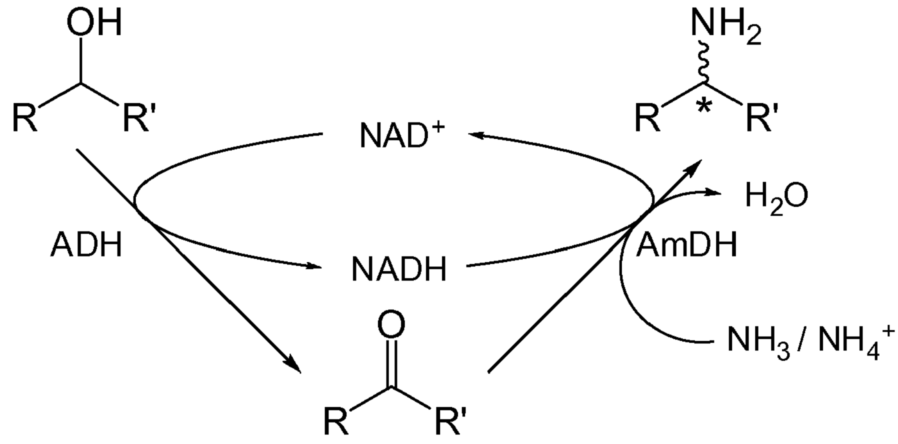


**Scheme 2. Hydrogen-borrowing biocatalytic amination of alcohols**

For example R = Alkyl-, Arylalkyl-; and R' = $CH_3$-, H-

In this redox self-sufficient network for the direct amination of an alcohol, wherein the source of nitrogen is ammonia, the only by-product is water. No other chemical species are required besides the ammonia. Therefore, in contrast to the previously described biocatalytic process (Scheme 1), the process of the invention is, surprisingly, truly catalytic. Furthermore this process possesses further surprising advantages, in particular a wider applicability since it allows for:

1) the amination of an enantiopure secondary alcohol with stereoinversion (*e.g.* from (*S*)-phenyl-2-propanol to (*R*)-phenyl-2-propylamine with >99% e.e.); (AA-ADH)
2) the amination of an enantiopure secondary alcohol with retention of configuration (*e.g.* from (*R*)-phenyl-2-propanol to (*R*)-phenyl-2-propylamine with >99% e.e); (LBv-ADH)
3) the asymmetric amination of racemic secondary alcohols (*e.g.* from rac-phenyl-2-propanol to (*R*)-phenyl-2-propylamine with >99% e.e
4) the amination of primary alcohols (*e.g.* from 2-phenyl-ethanol to 2-phenyl-ethylamine)

The combination of an alcohol dehydrogenase (ADH) with an amine dehydrogenase (AmDH) is a challenging task not described in the prior art so far. Various parameters alone or in combination have to be considered for ensuring optimum reaction conditions, and /or the compatibility between the two enzymes under the reaction conditions:

1) The amination requires an excess of ammonia / ammonium cations as the nitrogen source for driving the reaction to completion. Thus the concentration of ammonia / ammonium cations should be sufficiently high if a high conversion and elevated reaction rates have to be achieved, and without being at the same time detrimental for the stability of the two enzymes.
2) A reductive amination of a ketone with an AmDH was previously reported to be carried out at pH 9.6 (Abrahamson, M.J. et al, Angew. Chem. Int Ed,2012, 51, 3969). Surprisingly, the present invention shows that this is neither the optimal pH for the reductive amination nor for the amination of an alcohol through the ADH/AmDH cascade. In fact, a lower pH (8 - 8.7) allows for obtaining elevated conversions, mainly due to improved enzyme and cofactor stability.
3) This invention shows that the preferred AmDH enzyme is active under a wide range of pH. However, some ADHs are not stable at pH > 8.5 in presence of elevate concentrations of ammonia (e.g. 2 M).
4) The choice of the buffer is also important. The AmDH well-tolerates different buffers (*i.e.* ammonium chloride, formate, borate, citrate, acetate, oxalate, etc.). However, the ADHs employed in this invention are $Zn^{2+}$ dependent enzymes that, in some cases, possess a $Mg^{2+}$ as additional structural site; therefore strong chelators such as oxalate, citrate and acetate must be avoided as buffer salts.
5) His-terminal tags are convenient for easy and efficient enzyme purification. However, poly-histidine groups can coordinate metal cations such as $Zn^{2+}$. This fact has to be taken into account when setting up cascades *in vitro* involving metal-dependent enzymes

**Description of the figures:**

Figure 1 illustrates the influence of the pH and of the type of buffer on the relative conversion for the reductive amination using Ph-AmDH and stoichiometric NADH cofactor.

Figure 2 illustrates the results of a time study for the biocatalytic reductive amination catalysed by Ph-AmDH, catalytic NADH and using para-fluoro-phenylacetone (20 mM) and ammonia (727 mM) at pH 8.7.

Figure 3 shows the chemical formulae of ubstrate tested for the reductive amination using Ph-AmDH.

Figure 4 illustrates the amination reaction of (S)-phenyl-2-propanol (20 mM) to give (R)-phenyl-2-propylamine employing AA-ADH and Ph-AmDH in the presence of catalytic amounts of NAD+ (1 mM). Conversion for amine product (solid line), ketone intermediate (dotted line) and alcohol substrate (dashed line) was monitored during the course of time.

Figure 5 illustrates the amination reaction of (S)-phenyl-2-propanol (20 mM) to give (R)-phenyl-2-propylamine employing AA-ADH and Ph-AmDH in the presence of catalytic amounts of NAD+ (0.5 mM). Conversion for amine product (solid line), ketone intermediate (dotted line) and alcohol substrate (dashed line) was monitored during the time.

Figure 6 illustrates the amination reaction of (S)-phenyl-2-propanol (20 mM) to give (R)-phenyl-2-propylamine employing AA-ADH and Ph-AmDH in the presence of catalytic amounts of NAD+ (0.2 mM). Conversion for amine product (solid line), ketone intermediate (dotted line) and alcohol substrate (dashed line) was monitored during the time.

Figure 7 illustrates the amination reaction of (S)-phenyl-2-propanol (20 mM) to give (R)-phenyl-2-propylamine employing AA-ADH and Ph-AmDH in the presence of catalytic amounts of $NAD^+$ (0.1 mM). Conversion for amine product (solid line), ketone intermediate (dotted line) and alcohol substrate (dashed line) was monitored during the time.

Figure 8 shows the sequence alignment of *B. badius* Phenylalanine dehydrogenase (Ph-DH) (upper line) of Ph-AmDH mutant derived therefrom (middle line) and the consensus sequence thereof (lower line)

Figure 9 shows the sequence alignment of *B. stearothermophilus* LeuDH (upper line) of Leu-AmDH mutant derived therefrom (middle line) and the consensus sequence thereof (lower line)

**Detailed description of the invention**

**1. Specific embodiments**

The present invention relates to the following particular embodiments:

1. A biocatalytic method of preparing a hydrocarbyl amine, in particular aliphatic or aromatic amines, which method comprises:

a) the enzymatic oxidation of the corresponding primary or secondary alcohol of said amine in the presence of the cofactor $NAD(P)^+$, in particular $NAD^+$, and of an alcohol dehydrogenase (ADH) (E.C.1.1.1.x) to form the corresponding oxo compound of said alcohol, while the cofactor $NAD(P)^+$, in particular $NAD^+$, is reduced to form NAD(P)H, in particular NADH, and
b) the enzymatic reduction (reductive amination) of said oxo compound (i.e. carbonyl compound) in the presence of

i)) ammonia or an ammonia source, and
ii) an amine dehydrogenase (AmDH) (E.C.1.4.1.x.)

to form the desired amine,

while the cofactor $NAD(P)^+$, in particular $NAD^+$, is regenerated from NAD(P)H; , in particular NADH, and optionally c) isolating said amine.
In the case of asymmetric alcohol substrates the substrate may be in the prom of a mixture of stereoisomers or may be employed in stereoisomerically pure form.
Moreover, in the case of stereoismerically pure substrates the reaction product may be formed with stereoinversion ((R) -> (S) or (S) -> (R)) or retention of the configuration.
Depending on the type of alcohol (primary or secondary) different ADHs are preferably employed.
Preferably the enzymes as employed use the same cofactor, in particular NAD(H).

2. The method of embodiment 1, wherein steps a) and b) are performed simultaneously.

3. The method of one of the preceding embodiments, wherein said AmDH is obtained by protein engineering of an amino acid dehydrogenase, in particular oxidoreductases acting on the $CH-NH_2$ group of donor molecules (substrates) with NAD or NADP as acceptor, (E.C. 1.4.1.).

4. The method of one of the preceding embodiments, wherein said AmHD is a mutant of an amino acid dehydrogenase enzyme, which mutant has the ability to convert, in the presence of ammonia or an ammonia source and NAD(P)H, an oxo compound to the corresponding primary hydrocarbyl amine.

5. The method of embodiment 4 or 5, wherein said AmDH is a mutant of a phenylalanine dehydrogenase (Ph-AmDH) (E.C. 1.4.1.20) or a mutant of a leucine dehydrogenase (L-AmDH) (E.C. 1.4.1.9).

6. The method of embodiment 5, wherein said AmDH is selected from a

a) a mutant of a phenyl alanine dehydrogenase comprises an amino acid sequence of SEQ ID NO: 1, wherein said mutant comprises the double mutation K78S,N277L, and wherein said mutant has a sequence identity of at least 80 %, like 85, 90, 92, 95, 96, 97, 98 or 99 % sequence identity, to SEQ ID NO: 1, and
b) a mutant of a leucine dehydrogenase comprises an amino acid sequence of SEQ ID NO: 3, wherein said mutant comprises the four-fold mutation K68S,E114V,N262L,V291C, and wherein said mutant has a sequence identity of at least 80 %, like 85, 90, 92, 95, 96, 97, 98 or 99 % sequence identity, to SEQ ID NO: 3.

7. The method of one of the preceding embodiments wherein

a) said AmDH is a mutant of a phenyl alanine dehydrogenase and comprises an amino acid sequence of SEQ ID NO: 2, or of amino acid residues 21 to 400 of SEQ ID NO:2 and/or
b) said AmDH is a mutant of a leucine alanine dehydrogenase comprises an amino acid sequence of SEQ ID NO: 4,and/ or
c) said ADH is selected from AA-ADH comprising an amino acid sequence of SEQ ID NO:5 or an amino acid sequence having a sequence identity of at least 80 %, like 85, 90, 92, 95, 96, 97, 98 or 99 % sequence identity, to SEQ ID NO: 5; LBv-ADH comprising an amino acid sequence of SEQ ID NO:6 or an amino acid sequence having a sequence identity of at least 80 %, like 85, 90, 92, 95, 96, 97, 98 or 99 % sequence identity, to SEQ ID NO: 6; HL-ADH comprising an amino acid sequence of SEQ ID NO: 8 or an amino acid sequence having a sequence identity of at least 80 %, like 85, 90, 92, 95, 96, 97, 98 or 99 % sequence identity, to SEQ ID NO: 8; ADH-A comprising an amino acid sequence of SEQ ID NO: 9 or an amino acid sequence having a sequence identity of at least 80 %, like 85, 90, 92, 95, 96, 97, 98 or 99 % sequence identity, to SEQ ID NO: 9; or ADH-hT comprising an amino acid sequence of SEQ ID NO: 10 or an amino acid sequence having a sequence identity of at least 80 %, like 85, 90, 92, 95, 96, 97, 98 or 99 % sequence identity, to SEQ ID NO: 10.
For converting primary alcohols , preferably primary ADHs, like HL-ADH or ADH-ht are employed. For converting secondary alcohols, preferably secondary ADHs, like ADH-A, LBv.-ADH or AA-ADH are employed.

8. The method of one of the preceding embodiments wherein said amine is of the general formula I

$$(R_1)(R_2)HC-NH_2 \qquad (I)$$

wherein
$R_1$ and $R_2$ are identical or different and selected from H, alkyl, cycloalkyl, alkenyl, aryl, aralkyl, alkoxyalkyl, or aryloxyalkyl provided that $R_1$ and $R_2$ are not simultaneously H, and wherein aryl moiety optionally may be substituted.
Preferred products are:

a) Amines wherein one of the residues $R_1$ and $R_2$ is H and the other is selected from

- linear or branched alkyl, in particular linear or branched $C_1$-$C_{10}$ alkyl;
- optionally substituted aralkyl, in particular optionally substituted phenyl-$C_1$-$C_4$ alkyl, like optionally substituted phenyl-methyl;
- alkoxyalkyl, like $C_1$-$C_{10}$alkoxy-$C_1$-$C_4$ alkyl or like $C_1$-$C_6$alkoxy-methyl;
- optionally substituted aryloxyalkyl, like optionally substituted phenyloxy-$C_1$-$C_4$alkyl or optionally substituted phenyloxy-methyl;

b) Amines wherein $R_1$ and $R_2$ are the same or different and selected from

- linear or branched alkyl, in particular linear or branched $C_1$-$C_{10}$ alkyl, preferably methyl or ethyl;
- optionally substituted aralkyl, in particular optionally substituted phenyl-$C_1$-$C_4$alkyl, like optionally substituted phenyl-methyl;
- alkoxyalkyl, like $C_1$-$C_{10}$alkoxy-$C_1$-$C_4$alkyl or like $C_1$-$C_6$ alkoxy-methyl;
- optionally substituted aryloxyalkyl, like optionally substituted phenyloxy-$C_1$-$C_4$ alkyl or optionally substituted phenyloxy-methyl;

Particularly preferred amines are those wherein one of the residues $R_1$ and $R_2$ is methyl or ethyl and the other is selected from the above preferred meanings.
Optional substituents are preferably selected from Halogen, like F, Cl, Br, $C_1$-$C_4$ alkoxy, $C_1$-$C_4$ alkyl, optionally carrying a terminal or intrachain heteroatom, like-NH- or-$NH_2$, or being optionally substituted by halogen, like *e.g.* $CF_3$.
Particularly preferred substrates are the corresponding alcohols of the Oxo-compounds depicted in Fig.3.

9. The method of one of the preceding embodiments, which is performed in an aqueous reaction medium.

10. The method of one of the preceding embodiments wherein the method is performed in the presence of the set of required enzymes in isolated form, one or more recombinant microorganisms expressing at least one enzyme of the required set of enzymes or cell homogenates or cell lysates of one ore more microorganisms expressing the required set of enzymes or at least one of the enzyme of the required set of enzymes.

11. The method of one of the preceding embodiments wherein the alcohol substrates are selected from monovalent alcohols of the formula II:

$$(R_1)(R_2)HC\text{-}OH \qquad (II)$$

wherein
$R_1$ and $R_2$ are as defined above.

12. The method of one of the preceding embodiments further comprising isolating the produced amine from the reaction medium.

13. The method of one of the preceding embodiments, wherein the cofactor is employed in sub-stoichiometric, i.e. catalytic amounts.

14. The method of one of the preceding embodiments, wherein the reaction is performed in the presence of a, on a molar basis molar, excess of ammonia or of said ammonia source, calculated on mole of alcohol to be aminated. Preferably there is a 1 to 100-fold like 2 to 50- or 5 to 20-fold molar excess..

15. The method of one of the preceding embodiments wherein the reaction id performed at a pH in the range of less the 9.6, in particular at about 8 to 8.7.

16. The method of one of the preceding embodiments wherein the pH is controlled by means of a buffer which does not show metal chelating properties, i.e. which does not contain carboxylic acid residues,. I.e. oxalate, citrate or acetate buffers, are less suitable buffer substances..

17. The method of one of the preceding embodiments wherein the employed enzymes are NAD(H) dependent.

18. A recombinant expression vector carrying under the control of at least one regulatory element the coding sequence of at least one ADH and/or at least one AmDH as defined in anyone of the embodiments 1 to 7.

19. A recombinant microorganism carrying at least one expression vector according to embodiment 18 and functionally expressing said encoded enzymes.

20. The use of a recombinant expression vector of embodiment 18 or of a recombinant microorganism of embodiment 19 in a method as mentioned in anyone of the embodiments 1 to 17.

21. A bioreactor for performing a biocatalytic method of anyone of the embodiments 1 to 17 containing in a reaction medium at least one recombinant microorganism of embodiment 19, or at least one enzyme as defined in anyone of the embodiments 1 to 7 in free or immobilized form.

## 2. Explanation of particular terms

Unless otherwise stated the following meanings shall apply:

If an overall multi-enzyme redox process is "redox neutral", no external redox equivalents are required; and such a system is defined as redox self-sufficient.

The term "biocatalytic" or "enzymatically catalysed" method refers to any method performed in the presence of catalytic activity of an enzyme as defined herein, i.e. in the presence of isolated pure or crude enzyme or entire (growing, resting, inactivated or perforated) microbial cells containing or expressing such enzyme activity or a cell homogenate thereof, or combinations of whole cells expressing at least one intended enzyme activity and of isolated pure or crude enzyme preparations containing at least one other intended enzyme activity, so that the entire set of enzyme activities is presented by the combination.

The term "stereospecific" means that one out of several stereoisomers or enantiomers is formed by the enzyme in high enantiomeric excess or purity, of at least 90 %ee, preferably at least 95 %ee, in particular at least 98 %ee, or at least 99 %ee. the ee% value is calculated according to the following formula

$$ee\% = [X_A - X_B]/[X_A + X_B]*100,$$

wherein $X_A$ and $X_B$ refer to the molar fraction of enantiomer A or B, respectively.

The term "substantially pure" is meant to describe a molecule, which is homogeneous by one or more purity or homogeneity characteristics used by those of skill in the art.

A "substantially pure" protein or enzyme means that the desired purified protein is essentially free from contaminating cellular components, as evidenced by a single band following polyacrylamide-sodium dodecyl sulfate gel electrophoresis (SDS-PAGE). A "substantially pure" enzyme or protein will show constant and reproducible characteristics within standard experimental deviations for parameters such as the following: molecular mass, chromatographic migration, amino acid composition, amino acid sequence, blocked or unblocked N-terminus, HPLC elution profile, biological activity, and other such parameters. For example, a "substantially pure" protein or enzyme means that the desired purified protein is essentially free from contaminating cellular components, as evidenced by a single band following polyacrylamide-sodium dodecyl sulfate gel electrophoresis (SDS-PAGE).The term, however, is not meant to exclude artificial or synthetic mixtures of said enzyme or protein with other compounds. In addition, the term is not meant to exclude fusion proteins optionally isolated from a recombinant host, or fusions proteins with functional residues like histidin archer groups.

"Natural alpha amino acid residues" are derived from Ala, Arg, Asn, Asp, Cys, Gin, Glu, Gly, His, lie, Leu, Lys, Met, Phe, Ser, Thr, Trp, Tyr, Val, Pro.

Within chemical compounds as defined herein and in the attached claims the following meanings shall apply:

**Hydrocarbyl** can be interpreted widely and comprises straight-chain and branched hydrocarbon radicals, linked to one functional group, like OH or $NH_2$, In particular these functional groups are terminal groups. These residues are essentially composed of carbon and hydrogen atoms, which may optionally additionally comprise heteroatoms, for example O, N, NH, S, within their chain thereof, and may have a chain length of 1 to 50, 2 to 30 or 3 to 15 consecutive carbon atoms and optionally heteroatoms .

Hydrocarbyl especially represents residues and especially straight-chain or branched alkyl, alkenyl or alkinyl, optionally interrupted by one or more, for example 1, 2, 3, 4, 5, 6, 7, 8, 9 or 10 heteroatom groups such as -O- or-NH-, in particular-O-.

In particular hydrocarbyl may be understood as covering exclusively aromatic and aliphatic residues composed

of hydrogen and carbon atoms, in particular aryl, alkyl, alkenyl and alkinyl residues as defined below. and alke

**Alkyl as well as alkyl fragments of residues derived therefrom, as for example alkoxy,** represent saturated, linear or branched hydrocarbon chains having 1 to 4, 1 to 6, 1 to 8, 1 to 10, 1 to 22 or 1 to 30 carbon atoms, like

**$C_1$-$C_{10}$-Alkyl** like **$C_1$-$C_4$-Alkyl**, as for example methyl, ethyl, propyl, 1-methylethyl, butyl, 1-methyl-propyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl und 1-ethyl-2-methylpropyl.

**$C_1$-$C_{10}$-Alkoxy,** like **$C_1$-$C_4$-alkoxy,** as for example methoxy, ethoxy, n-propoxy, 1-methylethoxy, butoxy, 1-methylpropoxy, 2-methylpropoxy or 1,1-dimethylethoxy; as well as e.g. pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy;

**Hydroxy-$C_1$-$C_{10}$-alkyl, e.g. hydroxy-$C_1$-$C_6$-alkyl, or hydroxy-$C_1$-$C_4$-alkyl, or hydroxy-$C_4$-$C_{10}$-alkyl:** e.g. hydroxymethyl, 1- or 2-hydroxyethyl, 1-, 2- or 3-hydroxypropyl, 1-hydroxymethylethyl, 1-, 2-, 3- or 4-hydroxybutyl, 1-hydroxymethylpropyl and 2-hydroxymethylpropyl; or other hydroxy-substituted analogs of the above alkyl residues, such as in particular the meanings of R given in Table 4, e.g. 6-hydroxy-6-methylhept-1-yl.

**Alkenyl:** one-fold or multiple, in particular one-fold non-saturated, straight-chain or branched hydrocarbon residues having 2 to 4, 2 to 6, 2 to 8, or 2 to 10 carbon atoms and a double bond in any position, like C2-C6-alkenyl, as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

**Alkenyloxy:** oxygen-linked analogs of the above alkenyl groups, like corresponding $C_2$-$C_6$-alkenyloxy groups.

**Aryl:** mono- or polynuclear, preferably mono- or dinuclear, optionally substituted aromatic radicals having 6 to 20, such as e.g. 6 to 10, ring carbon atoms, such as e.g. phenyl, biphenyl, naphthyl such as 1- or 2-naphthyl, tetrahydronaphthyl, fluorenyl, indenyl and phenanthrenyl. The aryl radicals can optionally carry 1, 2, 3, 4, 5 or 6 identical or different substituents. In the case of a polynuclear aryl radical, at least one of the rings has aromatic character; however, it is also possible for several or all of the rings to have aromatic character.

**Arylalkyl:** the aryl-substituted analogs of the above alkyl radicals, where aryl likewise has the aforementioned meanings, such as e.g. phenyl-$C_1$-$C_4$-alkyl radicals selected from phenylmethyl or phenylethyl.

**Aryloxy:** the oxygen-linked analogs of the above optionally substituted aryl radicals.

**Cycloalkyl:** carbocyclic radicals having 3 to 20 carbon atoms, such as e.g. $C_3$-$C_{12}$-cycloalkyl, such as cyclopropyl, cyclobutyl, cyclopentyl, cyclohexyl, cycloheptyl, cyclooctyl, cyclononyl, cyclodecyl, cycloundecyl and cyclododecyl; preference is given to cyclopentyl, cyclohexyl, cycloheptyl, and also cyclopropylmethyl, cyclopropylethyl, cyclobutylmethyl, cyclobutylethyl, cyclopentylmethyl, cyclopentylethyl, cyclohexylmethyl or $C_3$-$C_7$-cycloalkyl, such as cyclopropyl, cyclobutyl, cyclopentyl, cyclohexyl, cycloheptyl, cyclopropylmethyl, cyclopropylethyl, cyclobutylmethyl, cyclopentylethyl, cyclohexylmethyl, where the bond to the radical of the molecule can be via any suitable carbon atom.

**Cycloalkenyl:** monocyclic, monounsaturated hydrocarbon groups having 5 to 8, preferably up to 6, carbon ring members, such as cyclopenten-1-yl, cyclopenten-3-yl, cyclohexen-1-yl, cyclohexen-3-yl and cyclohexen-4-yl;

**Optional substituents** may be selected from - COOH,-OH, -SH, -CN, amino, - $NO_2$, alkyl, or alkenyl, alkyl or alkenyl, alkoxy, halogenated alkyl; being as defined above.

### 3. Further embodiments of the invention

#### 3.1 Proteins according to the invention

The present invention is not limited to the specifically mentioned enzymes/proteins, but also extends to functional

equivalents thereof.

"Functional equivalents" or "analogs" or "functional mutations" of the concretely disclosed enzymes are, within the scope of the present invention, various polypeptides thereof, which moreover possess the desired biological function or activity, e.g. enzyme activity.

For example, "functional equivalents" means enzymes, which, in a test used for enzymatic activity, display at least a 1 to 10%, or at least 20%, or at least 50%, or at least 75%, or at least 90% higher or lower activity of an enzyme, as defined herein.

"Functional equivalents", according to the invention, also means in particular mutants, which, in at least one sequence position of the amino acid sequences stated above, have an amino acid that is different from that concretely stated, but nevertheless possess one of the aforementioned biological activities. "Functional equivalents" thus comprise the mutants obtainable by one or more, like 1 to 20, 1 to 15, 1 to 10 or 1 to 5, amino acid additions, substitutions, deletions and/or inversions, where the stated changes can occur in any sequence position, provided they lead to a mutant with the profile of properties according to the invention. Functional equivalence is in particular also provided if the reactivity patterns coincide qualitatively between the mutant and the unchanged polypeptide, i.e. if for example the same substrates are converted at a different rate. Examples of suitable amino acid substitutions are shown in the following table:

| Original residue | Examples of substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Functional equivalents" in the above sense are also "precursors" of the polypeptides described, as well as "functional derivatives" and "salts" of the polypeptides.

"Precursors" are in that case natural or synthetic precursors of the polypeptides with or without the desired biological activity.

The expression "salts" means salts of carboxyl groups as well as salts of acid addition of amino groups of the protein molecules according to the invention. Salts of carboxyl groups can be produced in a known way and comprise inorganic salts, for example sodium, calcium, ammonium, iron and zinc salts, and salts with organic bases, for example amines, such as triethanolamine, arginine, lysine, piperidine and the like. Salts of acid addition, for example salts with inorganic acids, such as hydrochloric acid or sulfuric acid and salts with organic acids, such as acetic acid and oxalic acid, are also covered by the invention.

"Functional derivatives" of polypeptides according to the invention can also be produced on functional amino acid side groups or at their N-terminal or C-terminal end using known techniques. Such derivatives comprise for example aliphatic esters of carboxylic acid groups, amides of carboxylic acid groups, obtainable by reaction with ammonia or with a primary or secondary amine; N-acyl derivatives of free amino groups, produced by reaction with acyl groups; or O-acyl derivatives of free hydroxy groups, produced by reaction with acyl groups.

"Functional equivalents" naturally also comprise polypeptides that can be obtained from other organisms, as well as naturally occurring variants. For example, areas of homologous sequence regions can be established by sequence comparison, and equivalent enzymes can be determined on the basis of the concrete parameters of the invention.

"Functional equivalents" also comprise fragments, preferably individual domains or sequence motifs, of the polypeptides according to the invention, which for example display the desired biological function.

"Functional equivalents" are, moreover, fusion proteins, which have one of the polypeptide sequences stated above or functional equivalents derived there from and at least one further, functionally different, heterologous sequence in functional N-terminal or C-terminal association (i.e. without substantial mutual functional impairment of the fusion protein parts). Non-limiting examples of these heterologous sequences are e.g. signal peptides, histidine anchors or enzymes.

"Functional equivalents" that are also included according to the invention are homologues of the concretely disclosed proteins. These possess percent identity values as stated above. Said values refer to the identity with the concretely disclosed amino acid sequences, and may be calculated according to the algorithm of Pearson and Lipman, Proc. Natl. Acad, Sci. (USA) 85(8), 1988, 2444-2448.

The percent identity values may also be calculated from BLAST alignments, algorithm blastp (protein-protein BLAST) or by applying the Clustal setting as given below. Typical percent identity values are for example in the range of 50 % or more, as for example at least 60, 70, 80, 85, 90, 91, 92, 93, 94, 95, 96, 97, 98 or 99 %.

A percentage identity of a homologous polypeptide according to the invention means in particular the percentage identity of the amino acid residues relative to the total length of one of the amino acid sequences concretely described herein.

In the case of a possible protein glycosylation, "functional equivalents" according to the invention comprise proteins of the type designated above in deglycosylated or glycosylated form as well as modified forms that can be obtained by altering the glycosylation pattern.

Such functional equivalents or homologues of the proteins or polypeptides according to the invention can be produced by mutagenesis, e.g. by point mutation, lengthening or shortening of the protein.

Such functional equivalents or homologues of the proteins according to the invention can be identified by screening combinatorial databases of mutants, for example shortening mutants. For example, a variegated database of protein variants can be produced by combinatorial mutagenesis at the nucleic acid level, e.g. by enzymatic ligation of a mixture of synthetic oligonucleotides. There are a great many methods that can be used for the production of databases of potential homologues from a degenerated oligonucleotide sequence. Chemical synthesis of a degenerated gene sequence can be carried out in an automatic DNA synthesizer, and the synthetic gene can then be ligated in a suitable expression vector. The use of a degenerated genome makes it possible to supply all sequences in a mixture, which code for the desired set of potential protein sequences. Methods of synthesis of degenerated oligonucleotides are known to a person skilled in the art (e.g. Narang, S.A. (1983) Tetrahedron 39:3; Itakura et al. (1984) Annu. Rev. Biochem. 53:323; Itakura et al. (1984) Science 198:1056; Ike et al. (1983) Nucleic Acids Res. 11:477).

In the prior art, several techniques are known for the screening of gene products of combinatorial databases, which were produced by point mutations or shortening, and for the screening of cDNA libraries for gene products with a selected property. These techniques can be adapted for the rapid screening of the gene banks that were produced by combinatorial mutagenesis of homologues according to the invention. The techniques most frequently used for the screening of large gene banks, which are based on a high-throughput analysis, comprise cloning of the gene bank in expression vectors that can be replicated, transformation of the suitable cells with the resultant vector database and expression of the combinatorial genes in conditions in which detection of the desired activity facilitates isolation of the vector that codes for the gene whose product was detected. Recursive Ensemble Mutagenesis (REM), a technique that increases the frequency of functional mutants in the databases, can be used in combination with the screening tests, in order to identify homologues (Arkin and Yourvan (1992) PNAS 89:7811-7815; Delgrave et al. (1993) Protein Engineering 6(3):327-331).

### 3.2 Coding nucleic acid sequences

The invention also relates to nucleic acid sequences that code for enzymes/proteins as defined herein.

The present invention also relates to nucleic acids with a certain degree of "identity" to the sequences specifically disclosed herein. "Identity" between two nucleic acids means identity of the nucleotides, in each case over the entire length of the nucleic acid.

For example the identity may be calculated by means of the Vector NTI Suite 7.1 program of the company Informax (USA) employing the Clustal Method (Higgins DG, Sharp PM. Fast and sensitive multiple sequence alignments on a microcomputer. Comput Appl. Biosci. 1989 Apr; 5(2): 151-1) with the following settings:
Multiple alignment parameters:

| | |
|---|---|
| Gap opening penalty | 10 |
| Gap extension penalty | 10 |

(continued)

| | |
|---|---|
| Gap separation penalty range | 8 |
| Gap separation penalty | off |
| % identity for alignment delay | 40 |
| Residue specific gaps | off |
| Hydrophilic residue gap | off |
| Transition weighing | 0 |

Pairwise alignment parameter:

| | |
|---|---|
| FAST algorithm | on |
| K-tuplesize | 1 |
| Gap penalty | 3 |
| Window size | 5 |
| Number of best diagonals | 5 |

Alternatively the identity may be determined according to Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, the web page: http://www.ebi.ac.uk/Tools/clustalw/index.html# and the following settings

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

All the nucleic acid sequences mentioned herein (single-stranded and double-stranded DNA and RNA sequences, for example cDNA and mRNA) can be produced in a known way by chemical synthesis from the nucleotide building blocks, e.g. by fragment condensation of individual overlapping, complementary nucleic acid building blocks of the double helix. Chemical synthesis of oligonucleotides can, for example, be performed in a known way, by the phosphoamidite method (Voet, Voet, 2nd edition, Wiley Press, New York, pages 896-897). The accumulation of synthetic and filling of gaps by means of the Klenow fragment of DNA polymerase and ligation reactions as well as general cloning techniques are described in Sambrook et al. (1989), see below.

The invention also relates to nucleic acid sequences (single-stranded and double-stranded DNA and RNA sequences, e.g. cDNA and mRNA), coding for one of the above polypeptides and their functional equivalents, which can be obtained for example using artificial nucleotide analogs.

The invention relates both to isolated nucleic acid molecules, which code for polypeptides or proteins according to the invention or biologically active segments thereof, and to nucleic acid fragments, which can be used for example as hybridization probes or primers for identifying or amplifying coding nucleic acids according to the invention.

The nucleic acid molecules according to the invention can in addition contain non-translated sequences from the 3' and/or 5' end of the coding genetic region.

The invention further relates to the nucleic acid molecules that are complementary to the concretely described nucleotide sequences or a segment thereof.

The nucleotide sequences according to the invention make possible the production of probes and primers that can be used for the identification and/or cloning of homologous sequences in other cellular types and organisms. Such probes or primers generally comprise a nucleotide sequence region which hybridizes under "stringent" conditions (see below) on at least about 12, preferably at least about 25, for example about 40, 50 or 75 successive nucleotides of a sense strand of a nucleic acid sequence according to the invention or of a corresponding antisense strand.

An "isolated" nucleic acid molecule is separated from other nucleic acid molecules that are present in the natural source of the nucleic acid and can moreover be substantially free from other cellular material or culture medium, if it is

being produced by recombinant techniques, or can be free from chemical precursors or other chemicals, if it is being synthesized chemically.

A nucleic acid molecule according to the invention can be isolated by means of standard techniques of molecular biology and the sequence information supplied according to the invention. For example, cDNA can be isolated from a suitable cDNA library, using one of the concretely disclosed complete sequences or a segment thereof as hybridization probe and standard hybridization techniques (as described for example in Sambrook, J., Fritsch, E.F. and Maniatis, T. Molecular Cloning: A Laboratory Manual. 2nd edition, Cold Spring Harbor Laboratory, Cold Spring Harbor Laboratory Press, Cold Spring Harbor, NY, 1989). In addition, a nucleic acid molecule comprising one of the disclosed sequences or a segment thereof, can be isolated by the polymerase chain reaction, using the oligonucleotide primers that were constructed on the basis of this sequence. The nucleic acid amplified in this way can be cloned in a suitable vector and can be characterized by DNA sequencing. The according to the invention can also be produced by standard methods of synthesis, e.g. using an automatic DNA synthesizer.

Nucleic acid sequences according to the invention or derivatives thereof, homologues or parts of these sequences, can for example be isolated by usual hybridization techniques or the PCR technique from other bacteria, e.g. via genomic or cDNA libraries. These DNA sequences hybridize in standard conditions with the sequences according to the invention.

"Hybridize" means the ability of a polynucleotide or oligonucleotide to bind to an almost complementary sequence in standard conditions, whereas nonspecific binding does not occur between non-complementary partners in these conditions. For this, the sequences can be 90-100% complementary. The property of complementary sequences of being able to bind specifically to one another is utilized for example in Northern Blotting or Southern Blotting or in primer binding in PCR or RT-PCR.

Short of the conserved regions are used advantageously for hybridization. However, it is also possible to use longer fragments of the nucleic acids according to the invention or the complete sequences for the hybridization. These standard conditions vary depending on the nucleic acid used (oligonucleotide, longer fragment or complete sequence) or depending on which type of nucleic acid - DNA or RNA - is used for hybridization. For example, the melting temperatures for DNA:DNA hybrids are approx. 10°C lower than those of DNA:RNA hybrids of the same length.

For example, depending on the particular nucleic acid, standard conditions mean temperatures between 42 and 58°C in an aqueous buffer solution with a concentration between 0.1 to 5 x SSC (1 X SSC = 0.15 M NaCl, 15 mM sodium citrate, pH 7.2) or additionally in the presence of 50% formamide, for example 42°C in 5 x SSC, 50% formamide. Advantageously, the hybridization conditions for DNA:DNA hybrids are 0.1 x SSC and temperatures between about 20°C to 45°C, preferably between about 30°C to 45°C. For DNA:RNA hybrids the hybridization conditions are advantageously 0.1 x SSC and temperatures between about 30°C to 55°C, preferably between about 45°C to 55°C. These stated temperatures for hybridization are examples of calculated melting temperature values for a nucleic acid with a length of approx. 100 nucleotides and a G + C content of 50% in the absence of formamide. The experimental conditions for DNA hybridization are described in relevant genetics textbooks, for example Sambrook et al., 1989, and can be calculated using formulae that are known by a person skilled in the art, for example depending on the length of the nucleic acids, the type of hybrids or the G + C content. A person skilled in the art can obtain further information on hybridization from the following textbooks: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

"Hybridization" can in particular be carried out under stringent conditions. Such hybridization conditions are for example described in Sambrook, J., Fritsch, E.F., Maniatis, T., in: Molecular Cloning (A Laboratory Manual), 2nd edition, Cold Spring Harbor Laboratory Press, 1989, pages 9.31-9.57 or in Current Protocols in Molecular Biology, John Wiley & Sons, N.Y. (1989), 6.3.1-6.3.6.

"Stringent" hybridization conditions mean in particular: Incubation at 42°C overnight in a solution consisting of 50% formamide, 5 x SSC (750 mM NaCl, 75 mM tri-sodium citrate), 50 mM sodium phosphate (pH 7.6), 5x Denhardt Solution, 10% dextran sulfate and 20 g/ml denatured, sheared salmon sperm DNA, followed by washing of the filters with 0.1 x SSC at 65°C.

The invention also relates to derivatives of the concretely disclosed or derivable nucleic acid sequences.

Thus, further nucleic acid sequences according to the invention can be derived from the sequences specifically disclosed herein and can differ from it by addition, substitution, insertion or deletion of individual or several nucleotides, and furthermore code for polypeptides with the desired profile of properties.

The invention also encompasses nucleic acid sequences that comprise so-called silent mutations or have been altered, in comparison with a concretely stated sequence, according to the codon usage of a special original or host organism, as well as naturally occurring variants, e.g. splicing variants or allelic variants, thereof.

It also relates to sequences that can be obtained by conservative nucleotide substitutions (i.e. the amino acid in question is replaced by an amino acid of the same charge, size, polarity and/or solubility).

The invention also relates to the molecules derived from the concretely disclosed nucleic acids by sequence polymorphisms. These genetic polymorphisms can exist between individuals within a population owing to natural variation. These natural variations usually produce a variance of 1 to 5% in the nucleotide sequence of a gene.

Derivatives of nucleic acid sequences according to the invention mean for example allelic variants, having at least 60% homology at the level of the derived amino acid, preferably at least 80% homology, quite especially preferably at least 90% homology over the entire sequence range (regarding homology at the amino acid level, reference should be made to the details given above for the polypeptides). Advantageously, the homologies can be higher over partial regions of the sequences.

Furthermore, derivatives are also to be understood to be homologues of the nucleic acid sequences according to the invention, for example animal, plant, fungal or bacterial homologues , shortened sequences, single-stranded DNA or RNA of the coding and noncoding DNA sequence. For example, homologues have, at the DNA level, a homology of at least 40%, preferably of at least 60%, especially preferably of at least 70%, quite especially preferably of at least 80% over the entire DNA region given in a sequence specifically disclosed herein.

Moreover, derivatives are to be understood to be, for example, fusions with promoters. The promoters that are added to the stated nucleotide sequences can be modified by at least one nucleotide exchange, at least one insertion, inversion and/or deletion, though without impairing the functionality or efficacy of the promoters. Moreover, the efficacy of the promoters can be increased by altering their sequence or can be exchanged completely with more effective promoters even of organisms of a different genus.

### 3.3 Preparation of functional mutants

The skilled reader is also aware of methods of generating functional mutants of particular sequences disclosed herein.

Depending on the technique applied, a skilled reader may generate arbitrary or directed mutations in genes or non-coding nucleic acid regions (which, for example, may be of importance for regulating gene expression) and, afterwards, may generate suitable gene libraries. The molecular biological method required therefore all well known in the art, and, for example, described by Sambrook and Russell, Molecular Cloning. 3. Edition, Cold Spring Harbor Laboratory Press 2001.

Methods of modifying genes and consequently of modifying the encoded proteins are well known to the skilled reader, as for example

- site-specific mutagenesis wherein single or multiple nucleotides of a gene are specifically replaced (Trower MK (Ed.) 1996; In vitro mutagenesis protocols. Humana Press, New Jersey),
- saturation mutagenesis, wherein at any position of a gene the codon of any amino acid may be exchanged or added (Kegler-Ebo DM, Docktor CM, DiMaio D (1994) Nucleic Acids Res 22:1593; Barettino D, Feigenbutz M, Valcarel R, Stunnenberg HG (1994) Nucleic Acids Res 22:541; Barik S (1995) Mol Biotechnol 3:1),
- error-prone polymerase chain reaction(PCR), wherein nucleotide sequences are mutated via the action of an incorrectly functioning DNA-polymerase (Eckert KA, Kunkel TA (1990) Nucleic Acids Res 18:3739);
- SeSaM method (Sequence Saturation Method), wherein preferred substitutions are avoided by the polymerase (Schenk et al., Biospektrum, Vol. 3, 2006, 277-279)
- Passaging of genes in mutator-strains, showing an increased occurrence of mutations of nucleotide sequences, for example in view of a defective DNA-repair mechanism (Greener A, Callahan M, Jerpseth B (1996) An efficient random mutagenesis technique using an E.coli mutator strain. In: Trower MK (Hrsg.) In vitro mutagenesis protocols. Humana Press, New Jersey), or
- DNA-Shuffling, wherein a pool of closely related genes is formed and digested and wherein the fragments are used as templates for a PCR reaction, and wherein full-length mosaic genes are formed (Stemmer WPC (1994) Nature 370:389; Stemmer WPC (1994) Proc Natl Acad Sci USA 91:10747).

By applying the so-called directed evolution technique (see for example Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) a skilled reader will be able to specifically prepare functional mutants in large scale. In a first step libraries of a specific protein are generated, for example, by applying any one of the above mentioned methods. Afterwards said libraries are expressed, for example by applying bacteria or phage display systems.

Those genes expressing functional mutants showing the desired feature profile may be selected and subjected to further mutation. The steps of mutation and selection or screening may be repeated iteratively until one of the obtained mutants shows the desired feature profile.

By the iterative approach a limited number of mutations, as for example 1 to 5 mutations, may be performed

and their influence on the enzyme feature at issue may be evaluated and further improved mutants may be selected stepwise. Said selected mutant may then be subjected to a further mutation in substantially the same may. The number of single mutants to be evaluated may be reduced significantly in this way.

The teaching of the present invention provide important information as regards structure and sequence of the enzyme/ protein at issue, based on which it should be possible to generate further enzymes/proteins with the desired modified feature profile. In particular, so-called hot spots, i.e. sequence regions may be defined, which potentially may be suited for further mutation in order to modify or generate a desired feature of the enzyme/protein.

### 3.4 Constructs used according to the invention

The invention also relates to expression constructs, containing, under the genetic control of regulatory nucleic acid sequences, a nucleic acid sequence coding for a polypeptide or fusion protein according to the invention; as well as vectors comprising at least one of these expression constructs.

"Expression unit" means, according to the invention, a nucleic acid with expression activity, which comprises a promoter as defined herein and, after functional association with a nucleic acid that is to be expressed or a gene, regulates the expression, i.e. the transcription and the translation of this nucleic acid or of this gene. In this context, therefore, it is also called a "regulatory nucleic acid sequence". In addition to the promoter, other regulatory elements may be present, e.g. enhancers.

"Expression cassette" or "expression construct" means, according to the invention, an expression unit, which is functionally associated with the nucleic acid that is to be expressed or the gene that is to be expressed. In contrast to an expression unit, an expression cassette thus comprises not only nucleic acid sequences which regulate transcription and translation, but also the nucleic acid sequences which should be expressed as protein as a result of the transcription and translation.

The terms "expression" or "overexpression" describe, in the context of the invention, the production or increase of intracellular activity of one or more enzymes in a microorganism, which are encoded by the corresponding DNA. For this, it is possible for example to insert a gene in an organism, replace an existing gene by another gene, increase the number of copies of the gene or genes, use a strong promoter or use a gene that codes for a corresponding enzyme with a high activity, and optionally these measures can be combined.

Preferably such constructs according to the invention comprise a promoter 5'-upstream from the respective coding sequence, and a terminator sequence 3'-downstream, and optionally further usual regulatory elements, in each case functionally associated with the coding sequence.

A "promoter", a "nucleic acid with promoter activity" or a "promoter sequence" mean, according to the invention, a nucleic acid which, functionally associated with a nucleic acid that is to be transcribed, regulates the transcription of this nucleic acid.

"Functional" or "operative" association means, in this context, for example the sequential arrangement of one of the nucleic acids with promoter activity and of a nucleic acid sequence that is to be transcribed and optionally further regulatory elements, for example nucleic acid sequences that enable the transcription of nucleic acids, and for example a terminator, in such a way that each of the regulatory elements can fulfill its function in the transcription of the nucleic acid sequence. This does not necessarily require a direct association in the chemical sense. Genetic control sequences, such as enhancer sequences, can also exert their function on the target sequence from more remote positions or even from other DNA molecules. Arrangements are preferred in which the nucleic acid sequence that is to be transcribed is positioned behind (i.e. at the 3' end) the promoter sequence, so that the two sequences are bound covalently to one another. The distance between the promoter sequence and the nucleic acid sequence that is to be expressed transgenically can be less than 200 bp (base pairs), or less than 100 bp or less than 50 bp.

Apart from promoters and terminators, examples of other regulatory elements that may be mentioned are targeting sequences, enhancers, polyadenylation signals, selectable markers, amplification signals, replication origins and the like. Suitable regulatory sequences are described for example in Goeddel, Gene Expression Technology: Methods in Enzymology 185, Academic Press, San Diego, CA (1990).

Nucleic acid constructs according to the invention comprise in particular sequences selected from those, specifically mentioned herein or derivatives and homologues thereof, as well as the nucleic acid sequences that can be derived from amino acid sequences specifically mentioned herein which are advantageously associated operatively or functionally with one or more regulating signal for controlling, e.g. increasing, gene expression.

In addition to these regulatory sequences, the natural regulation of these sequences can still be present in front of the actual structural genes and optionally can have been altered genetically, so that natural regulation is switched off and the expression of the genes has been increased. The nucleic acid construct can also be of a simpler design, i.e. without any additional regulatory signals being inserted in front of the coding sequence and without removing the natural promoter with its regulation. Instead, the natural regulatory sequence is silenced so that regulation no longer takes place and gene expression is increased.

A preferred nucleic acid construct advantageously also contains one or more of the aforementioned enhancer sequences, functionally associated with the promoter, which permit increased expression of the nucleic acid sequence. Additional advantageous sequences, such as other regulatory elements or terminators, can also be inserted at the 3' end of the DNA sequences. One or more copies of the nucleic acids according to the invention can be contained in the construct. The construct can also contain other markers, such as antibiotic resistances or auxotrophy-complementing genes, optionally for selection on the construct.

Examples of suitable regulatory sequences are contained in promoters such as cos-, tac-, trp-, tet-, trp-tet-, lpp-, lac-, lpp-lac-, $lacI^{q-}$, T7-, T5-, T3-, gal-, trc-, ara-, rhaP ($rhaP_{BAD}$)SP6-, lambda-$P_R$- or in the lambda-$P_L$ promoter, which find application advantageously in Gram-negative bacteria. Other advantageous regulatory sequences are contained for example in the Gram-positive promoters ace, amy and SPO2, in the yeast or fungal promoters ADC1, MFalpha, AC, P-60, CYC1, GAPDH, TEF, rp28, ADH. Artificial promoters can also be used for regulation.

For expression, the nucleic acid construct is inserted in a host organism advantageously in a vector, for example a plasmid or a phage, which permits optimum expression of the genes in the host. In addition to plasmids and phages, vectors are also to be understood as meaning all other vectors known to a person skilled in the art, e.g. viruses, such as SV40, CMV, baculovirus and adenovirus, transposons, IS elements, phasmids, cosmids, and linear or circular DNA. These vectors can be replicated autonomously in the host organism or can be replicated chromosomally. These vectors represent a further embodiment of the invention.

Suitable plasmids are, for example in *E. coli,* pLG338, pACYC184, pBR322, pUC18, pUC19, pKC30, pRep4, pHS1, pKK223-3, pDHE19.2, pHS2, pPLc236, pMBL24, pLG200, pUR290, $pIN-III^{113}$-B1, λgt11 or pBdCl; in nocardioform actinomycetes pJAM2; in *Streptomyces* pIJ101, pIJ364, pIJ702 or pIJ361; in bacillus pUB110, pC194 or pBD214; in *Corynebacterium* pSA77 or pAJ667; in fungi pALS1, pIL2 or pBB116; in yeasts 2alphaM, pAG-1, YEp6, YEp13 or pEMBLYe23 or in plants pLGV23, pGHlac', pBIN19, pAK2004 or pDH51. The aforementioned plasmids represent a small selection of the possible plasmids. Other plasmids are well known to a person skilled in the art and will be found for example in the book Cloning Vectors (Eds. Pouwels P.H. et al. Elsevier, Amsterdam-New York-Oxford, 1985, ISBN 0 444 904018). Further suitable plasmids are also mentioned in the experimental part.

In a further embodiment of the vector, the vector containing the nucleic acid construct according to the invention or the nucleic acid according to the invention can be inserted advantageously in the form of a linear DNA in the microorganisms and integrated into the genome of the host organism through heterologous or homologous recombination. This linear DNA can comprise a linearized vector such as plasmid or just the nucleic acid construct or the nucleic acid according to the invention.

For optimum expression of heterologous genes in organisms, it is advantageous to alter the nucleic acid sequences in accordance with the specific codon usage employed in the organism. The codon usage can easily be determined on the basis of computer evaluations of other, known genes of the organism in question.

The production of an expression cassette according to the invention is based on fusion of a suitable promoter with a suitable coding nucleotide sequence and a terminator signal or polyadenylation signal. Common recombination and cloning techniques are used for this, as described for example in T. Maniatis, E.F. Fritsch and J. Sambrook, Molecular Cloning: A Laboratory Manual, Cold Spring Harbor Laboratory, Cold Spring Harbor, NY (1989) as well as in T.J. Silhavy, M.L. Berman and L.W. Enquist, Experiments with Gene Fusions, Cold Spring Harbor Laboratory, Cold Spring Harbor, NY (1984) and in Ausubel, F.M. et al., Current Protocols in Molecular Biology, Greene Publishing Assoc. and Wiley Interscience (1987).

The recombinant nucleic acid construct or gene construct is inserted advantageously in a host-specific vector for expression in a suitable host organism, to permit optimum expression of the genes in the host. Vectors are well known to a person skilled in the art and will be found for example in "Cloning Vectors" (Pouwels P.H. et al., Publ. Elsevier, Amsterdam-New York-Oxford, 1985).

### 3.5 Hosts that can be used according to the invention

Depending on the context, the term "microorganism" means the starting microorganism (wild-type) or a genetically modified microorganism according to the invention, or both.

The term "wild-type" means, according to the invention, the corresponding starting microorganism, and need not necessarily correspond to a naturally occurring organism.

By means of the vectors according to the invention, recombinant microorganisms can be produced, which have been transformed for example with at least one vector according to the invention and can be used for the fermentative production according to the invention.

Advantageously, the recombinant constructs according to the invention, described above, are inserted in a suitable host system and expressed. Preferably, common cloning and transfection methods that are familiar to a person skilled in the art are used, for example co-precipitation, protoplast fusion, electroporation, retroviral transfection and the like, in order to secure expression of the stated nucleic acids in the respective expression system. Suitable systems are

described for example in Current Protocols in Molecular Biology, F. Ausubel et al., Publ. Wiley Interscience, New York 1997, or Sambrook et al. Molecular Cloning: A Laboratory Manual. 2nd edition, Cold Spring Harbor Laboratory, Cold Spring Harbor Laboratory Press, Cold Spring Harbor, NY, 1989.

The host organism or host organisms according to the invention preferably contain at least one of the nucleic acid sequences, nucleic acid constructs or vectors described in this invention, which code for an enzyme activity according to the above definition.

### 3.6 Enzymatic production of products of the invention

The biocatalytic conversion reaction may be run in a liquid, preferably aqueous reaction medium, containing the substrate to be converted (primary or secondary alcohol) the required enzyme activities (amine dehydrogenase and primary or secondary ADH adapted to the type of alcohol substrate), the supplemented by Cofactor, ammonia or ammonium source, buffer, and optionally further ingredients, , as for example suitable for stabilizing enzyme activity.

The reaction mixture may be homogenous or non-homogenous, preferably homogenous.

An organic co-solvent may be added in a suitable proportion of from 0,1 to 80 vol.-% where appropriate. Optionally the substrate may be pre-dissolved in the organic co-solvent and then be added to the aqueous medium.

The reaction mixture may be agitated or shaken at a suitable intensity, at a suitable temperature in the range of 10 to 80, 10 to 50, or 25 to 40 like 25 to 30 °C for a suitable period of time. After sufficient reaction time, as for example 1 to 120 h or 10 to 24 h the products may be extracted.

Aliquots of the required enzyme as defined above are provided in dissolved or dispersed, for example immobilized form, in proper enzyme concentrations and activities and in proper relative proportions for performing the biotransformations.

A source of ammonia, as for example an ammonium salt, like ammonium chloride, will be present (in excess amount .i.e. more than equimolar amount to the substrate.) which may serve as a buffer itself, as for example ammonia chloride buffer.

Ideally, substantially only ammonia, an ammonia salt are added to the enzymes the cofactor and the substrate.

However, the reaction may also be run in a buffered system. Thus, the reaction can also be performed in buffered reaction medium containing a suitable buffer, as for example phosphate or borate buffer, preferably in a pH range of about 7 to about 10, like 8 to 9.

Further reaction details may be taken form the experimental section

### 3.7 Production methods of amines of the invention

The invention also relates to methods for the biocatalytic or fermentative (whole cell) production of amines, in particular of formula (I).

The recombinant microorganisms expressing/ carrying the required enzyme activities as used according to the invention can be cultivated continuously or discontinuously in the batch process or in the fed batch or repeated fed batch process. A review of known methods of cultivation will be found in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium that is to be used must satisfy the requirements of the particular strains in an appropriate manner. Descriptions of culture media for various microorganisms are given in the handbook "Manual of Methods for General Bacteriology" of the American Society for Bacteriology (Washington D. C., USA, 1981).

These media that can be used according to the invention generally comprise one or more sources of carbon, sources of nitrogen, inorganic salts, vitamins and/or trace elements in the case of a fermentative production.

Preferred sources of carbon are sugars, such as mono-, di- or polysaccharides. Very good sources of carbon are for example glucose, fructose, mannose, galactose, ribose, sorbose, ribulose, lactose, maltose, sucrose, raffinose, starch or cellulose. Sugars can also be added to the media via complex compounds, such as molasses, or other by-products from sugar refining. It may also be advantageous to add mixtures of various sources of carbon. Other possible sources of carbon are oils and fats such as soybean oil, sunflower oil, peanut oil and coconut oil, fatty acids such as palmitic acid, stearic acid or linoleic acid, alcohols such as glycerol, methanol or ethanol and organic acids such as acetic acid or lactic acid.

Sources of nitrogen are usually organic or inorganic nitrogen compounds or materials containing these compounds. Examples of sources of nitrogen include ammonia gas or ammonium salts, such as ammonium sulfate, ammonium chloride, ammonium phosphate, ammonium carbonate or ammonium nitrate, nitrates, urea, amino acids or complex sources of nitrogen, such as corn-steep liquor, soybean flour, soybean protein, yeast extract, meat extract and others. The sources of nitrogen can be used separately or as a mixture.

Inorganic salt compounds that may be present in the media comprise the chloride, phosphate or sulfate salts

of calcium, magnesium, sodium, cobalt, molybdenum, potassium, manganese, zinc, copper and iron.

Inorganic sulfur-containing compounds, for example sulfates, sulfites, dithionites, tetrathionates, thiosulfates, sulfides, but also organic sulfur compounds, such as mercaptans and thiols, can be used as sources of sulfur.

Phosphoric acid, potassium dihydrogenphosphate or dipotassium hydrogenphosphate or the corresponding sodium-containing salts can be used as sources of phosphorus.

Chelating agents can be added to the medium, in order to keep the metal ions in solution. Especially suitable chelating agents comprise dihydroxyphenols, such as catechol or protocatechuate, or organic acids, such as citric acid.

The fermentation media used according to the invention may also contain other growth factors, such as vitamins or growth promoters, which include for example biotin, riboflavin, thiamine, folic acid, nicotinic acid, pantothenate and pyridoxine. Growth factors and salts often come from complex components of the media, such as yeast extract, molasses, corn-steep liquor and the like. In addition, suitable precursors can be added to the culture medium. The precise composition of the compounds in the medium is strongly dependent on the particular experiment and must be decided individually for each specific case. Information on media optimization can be found in the textbook "Applied Microbiol. Physiology, A Practical Approach" (Publ. P.M. Rhodes, P.F. Stanbury, IRL Press (1997) p. 53-73, ISBN 0 19 963577 3). Growing media can also be obtained from commercial suppliers, such as Standard 1 (Merck) or BHI (Brain heart infusion, DIFCO) etc.

All components of the medium are sterilized, either by heating (20 min at 2.0 bar and 121°C) or by sterile filtration. The components can be sterilized either together, or if necessary separately. All the components of the medium can be present at the start of growing, or optionally can be added continuously or by batch feed.

The temperature of the culture is normally between 15°C and 45°C, preferably 25°C to 40°C and can be kept constant or can be varied during the experiment. The pH value of the medium should be in the range from 5 to 8.5, preferably around 7.0. The pH value for growing can be controlled during growing by adding basic compounds such as sodium hydroxide, potassium hydroxide, ammonia or ammonia water or acid compounds such as phosphoric acid or sulfuric acid. Antifoaming agents, e.g. fatty acid polyglycol esters, can be used for controlling foaming. To maintain the stability of plasmids, suitable substances with selective action, e.g. antibiotics, can be added to the medium. Oxygen or oxygen-containing gas mixtures, e.g. the ambient air, are fed into the culture in order to maintain aerobic conditions. The temperature of the culture is normally from 20°C to 45°C. Culture is continued until a maximum of the desired product has formed. This is normally achieved within 10 hours to 160 hours.

The cells can be disrupted optionally by high-frequency ultrasound, by high pressure, e.g. in a French pressure cell, by osmolysis, by the action of detergents, lytic enzymes or organic solvents, by means of homogenizers or by a combination of several of the methods listed.

The enzyme activities required for performing the intended biotransformation) may be expressed, i.e. provided by one singe or two or more, in particular two or three recombinant microorganisms.

Vectors and methods adapted to the preparation of recombinant microorganisms carrying and expressing one or more, or even all enzyme activities required for performing the method of the invention are known in the art e.g. for one gene: pET-system, Novagen or pBAD-system, Invitrogen; Plasmid-system for two genes e.g. pACYC-Duet1, Novagen)

In an alternative, more preferred, embodiment the production method of the invention is not performed as classical fermentative process. Rather, the (recombinant) cells carrying (expressing) the required enzyme activities (at least one, or preferably all the enzyme activities required) may be contacted with a reaction medium containing the required substrate(s) and co-substrates like NAD(H) and/or NADP(H). Said medium may also contain conventional additives supporting the intended production process, like buffer(s), in order to control the pH of the reaction medium during the course of the process. The cells may be used as such (as obtained from a cell culture) or may be pre-treated in order to increase permeability of the cells for substrate, co-substrate and product molecules. For example, cells may be subjected to a temperature treatment (as for example, optionally repeated, freeze/thaw cycles and/or storage at ambient temperature in order to achieve the desired permeability).

Suitable protocols for performing such processes are known to a skilled reader.

### 3.8 Enzyme immobilization

If an enzyme as sued according to the invention is, immobilised, it is attached to an inert carrier. Suitable carrier materials are known in the art and are, e.g., disclosed in EP-A-1149849, EP-A-1 069 183 and DE-OS 100193773 as well as the literature references cited therein (all of which are specifically enclosed with regard to carrier materials). Examples for suitable carrier materials are clays, clay minerals such as kaolinite, diatomeceous erth, perlite, silica, alumina, sodium carbonate, calcium carbonate, cellulose powder, anion exchanger materials, synthetic polymers, such as polystyrene, acrylic resins, phenol formaldehyde resins, polyurethanes and polyolefines, such as polyethylene and polypropylene. For preparing carrier-bound enzymes the carrier materials usually are used in the form of fine powders, wherein porous forms are preferred. The particle size of the carrier material usually does not exceed 5 mm, in particular

2 mm. In case the at least one enzyme is present in a whole-cell-preparation, said whole-cell-preparation may be present in a free or immobilised form. Suitable carrier materials are e.g. Ca-alginate or Carrageenan. Enzymes as well as cells may directly be linked by glutaraldehyde. A wide range of immobilisation methods is known in the art (e.g. J. Lalonde and A. Margolin "Immobilization of Enzymes" in K. Drauz und H. Waldmann, Enzyme Catalysis in Organic Synthesis 2002, Vol.III, 991-1032, Wiley-VCH, Weinheim).

**3.9 Product isolation**

The methodology of the present invention can further include a step of recovering compounds as produced according to the invention. The term "recovering" includes extracting, harvesting, isolating or purifying the compound from culture or reaction media. Recovering the compound can be performed according to any conventional isolation or purification methodology known in the art including, but not limited to, treatment with a conventional resin (e.g., anion or cation exchange resin, non-ionic adsorption resin, etc.), treatment with a conventional adsorbent (e.g., activated charcoal, silicic acid, silica gel, cellulose, alumina, etc.), alteration of pH, solvent extraction (e.g., with a conventional solvent such as an alcohol, ethyl acetate, hexane and the like), distillation, dialysis, filtration, concentration, crystallization, recrystallization, pH adjustment, lyophilization and the like.

**3.10 Particular Amine Dehydrogenases**

Two particular amine dehydrogenases are preferably employed:L-AmDH and Ph-AmDH.

**a) L-AmDH:**

The L-AmDH was originated from the leucine dehydrogenase from Bacillus stearothermophilus (also called as Geobacillus stearothermophilus). The sequence of the wild-type enzyme has been published in the following paper:

S. Nagata, K. Tanizawa, N. Esaki, Y. Sakamoto, T. Ohshima, H. Tanaka, K. Soda, Biochemistry 1988, 27, 9056 - 9062.

Regarding to the sequence of the wild-type leucine dehydrogenase from Bacillus stearothermophilus, the following mutations were applied to obtain the L-AmDH variant:

K68S - E114V - N262L - V291 C

Since the crystal structure of the wild-type leucine dehydrogenase from Bacillus stearothermophilus is not available, the function of the mutations can be assigned by studying the crystal structure of an analogous enzyme: the phenylalanine dehydrogenase from Rhodococcus sp. M4. The crystal structure of the phenylalanine dehydrogenase from Rhodococcus sp. M4 is available in the apo as well as in the holo form (PDB: 1C1D and 1 BW9).

All the four amino acid residues, which were mutated, are located in the active site of the enzyme. The lysine and the asparagine residues (K68 and N262, respectively, in the sequence of the leucine dehydrogenase from Bacillus stearothermophilus) directly interact with the two oxygen atoms belonging to the carboxylic moiety of the $\alpha$-ketoacid substrate. Hence, K68 and N262 play a crucial role in the binding of the substrate via their charged side chains. Starting from the scaffold of the leucine dehydrogenase from Bacillus stearothermophilus, these two positions were mutated first as follows: K68S and N262L. Hence two charged amino acid residues were replaced with other non-charged, more hydrophobic residues such as a serine and a leucine. The new introduced residues permit hydrophobic interactions with non-natural substrates, lacking of the carboxylic moiety. As a consequence the L-AmDH accepts preferentially methyl isobutyl ketone, which corresponds to the substitution of the carboxylic moiety of the natural substrate with a methyl group. The third mutation involves the residue V291 of the leucine dehydrogenase from Bacillus stearothermophilus. This residue must be related to the proline of the wild-type phenylalanine dehydrogenase from Rhodococcus sp. M4. Thus V291 must be involved in the binding of the substrate from the opposite side respect to the position of K68 and N262 in the active site. From literature data, it seems that the V291 C mutation helps to reduce the Km value for methyl isobutyl ketone, without affecting the kcat value. The fourth mutation involves the residue E114, which is changed into a valine. This position corresponds to a proline in the homologue phenylalanine dehydrogenase from Rhodococcus sp. M4. The influence of this mutation is difficult to rationalise on the base of the crystal structure of the homologous enzyme, since the residue E114 must be positioned significantly apart from the substrate. From literature data, it seems that this mutation might contribute to increase the kcat for methyl isobutyl ketone (approximately 2 orders of magnitude). However, synergistic effects of the neighbouring residues must also play a crucial role. The change of substrate acceptance is illustrated by the following scheme :

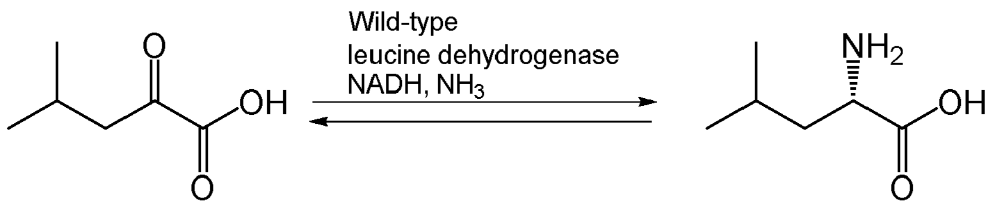


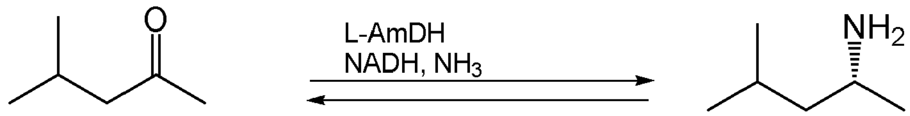


**b) Ph-AmDH**

The other preferred enzyme is the Ph-AmDH originated from the phenylalanine dehydrogenase from *Bacillus badius.* The sequence of the wild-type enzyme is reported in the literature:

A. Yamada, T. Dairi, Y. Ohno, X.-L. Huang, Y. Asano, Biosci. Biotech. Biochem. 1995, 59 (10), 1994 - 1995.

Regarding to the sequence of the wild-type phenylalanine dehydrogenase, the following mutations were applied to obtain the Ph-AmDH variant:

K78S - N277L

The phenylalanine dehydrogenase from Bacillus badius shares a relevant sequence similarity to the leucine dehydrogenase from Bacillus stearothermophilus (48% identity, 66% similarity). Therefore, the highly conserved residues K78 and N277 are also involved in the binding of the carboxylic moiety of the $\alpha$-ketoacid of the natural substrate. As previously described in the case of L-AmDH, the two synergic mutations of the lysine (K78) into serine and of the asparagine (N277) into a leucine produced the best variant for the target non-natural substrate (phenylacetone and derivatives thereof). No further mutations were required in this case in order to increase the enzyme activity. The change of substrate acceptance is illustrated by the following scheme :

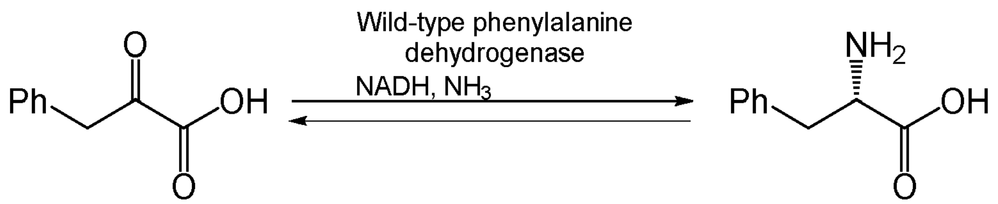


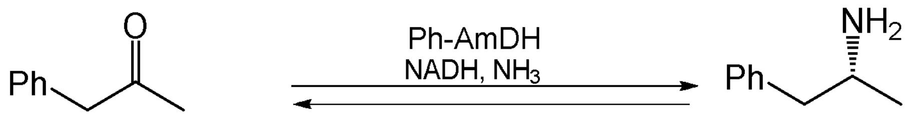


**Experimental Part**

Unless otherwise stated the following experiments have been performed by applying standard equipment, methods, chemicals, and biochemicals as used in genetic engineering, fermentative production of chemical compounds by cultivation of microorganisms and in the analysis and isolation of products. See also Sambrook et al, and Chmiel et al as cited herein above.

## Materials and methods

### a) General Methods

Protein concentrations were determined using the Uptima BC assay protein purification kit (Interchim, Montlucon, France) with bovine serum albumin as standard. SDS-PAGE was done in a Mini-Sub Cell GT (BioRad, Munich). Proteins were stained with SimplyBlue™ Safe Stain (Invitrogen, Carlsbad, CA, USA).

The enantiomeric excess of the amine was analyzed by GC .

### b) Chemicals

All chemicals were purchased from Fluka-Sigma-Aldrich (Munich, Germany) or Acros Organics (Geel, Belgium). BDG-Aldehyde and BDG-Amine were provided by BASF.

### c) Enzymes

**GDH:** The enzyme was purchased from Codexis. The reaction, which is used in the following experiments - the oxidation of glucose to gluconolactone, to simultaneously reduce NAD to NADH - is carried out with any enzyme of this type. Alternatively, for the NAD (P) H regeneration, the GDH of Bacillus subtilis (Lampel, K.A. et al., Journal of Bacteriology, 1986, 166, (1), 238) may be used. This amino acid sequence is deposited in GenBank under AAA22463.1. (SEQ ID NO:7)

All proteins were produced recombinantly in E. coli and optionally may carry for easier isolation a His-tag:

**Ph-AmDH (SEQ ID NO:2):** Muteine of Phenylalanine Dehydrogenase from *B. badius* as described by Abrahamson, M.J. et al., Advanced Synthesis and Catalysis, 2013, 355, (9), 1780-1786 and Abrahamson, M.J. et al,., Angew. Chem. Int Ed,2012, 51, 3969. The wild-type protein (SEQ ID NO:1) is also described by in Asano, Y. et al., European Journal of Biochemistry, 1987, 188, (1), 153.

**L-AmDH (SEQ ID NO:4):** Muteine of Leucine Dehydrogenase from *Geobacillus stearothermophilus*

The L-AmDH originated from the leucine dehydrogenase from *Geobacillus stearothermophilus.* The sequence of the wild-type enzyme has been published in the following paper: S. Nagata, K. Tanizawa, N. Esaki, Y. Sakamoto, T. Ohshima, H. Tanaka, K. Soda, Biochemistry 1988, 27, 9056 - 9062. Regarding to the sequence of the wild-type leucine dehydrogenase from *Geobacillus stearothermophilus,* the following mutations were applied to obtain the L-AmDH variant: K68S - E114V - N262L - V291C

L-AmDH and Ph-AmDH genes were custom-synthesized (cf. Carlson, R., Nat Biotechnol. 2009, 27, 1091) and cloned into the respective expression vectors.

**AA-ADH (SEQ ID NO:5):** see WO 2005/108590 der BASF SE or Höffken, H.W. et al., Biochemistry, 2006, 45, (1), 82.

**LBv-ADH (SEQ ID NO:6).** The mutant as used here is literature-known:: Schlieben, N.H. et al., Journal of Molecular Biology, 2005, 349, (4), 801. The biocatalyst in its wild-type form is described in DE 19610984 A1.

**HL-ADH (SEQ ID NO:8):** Isoform E was used, which, contrary to Isoform S reacts with ethanol but not with steroids (see Park, H.D. et al J. boil. Chem. 1991 266, (20) 13296)

**ADH-A (SEQ ID NO:9) :** see Kosjek et al., Biotechnol. Bioeng., (2004) 86, (1) 55; and US 7,569,375

**(ADH-hT):** see R. Cannio,M. Rossi, S. Bartolucci, Eur. J. Biochem. 1994, 222, 345 - 352; b) X. Zhang, T. C. Bruice, Biochemistry 2007, 46, 837 -843

### d) Cloning of genes

Routine manipulation of DNA, PCR and construction of recombinant plasmids were performed as described in (Sambrook J & Russell DW (2001) Molecular Cloning: a Laboratory Manual, 3rd edn. Cold Spring. Harbor Laboratory Press, Cold Spring Harbor, NY).

## Example 1: Enzyme preparations

### 1.1 Enzymes for the oxidative step (Alcohol oxidation)

The oxidative step as exemplified is performed by alcohol dehydrogenases which are NADH-dependent. NADPH-dependent alcohol dehydrogenases (ADH) cannot be applied because the coupled second reductive step of the exemplified reaction cascade is catalyzed by amine dehydrogenases (AmDH), which are strictly NADH-dependent.

The oxidation of primary alcohols is performed by a single primary alcohol dehydrogenase. Suitable primary

alcohol dehydrogenases are the alcohol dehydrogenase from horse liver (HL-ADH) (a) D. H. Park, B. V. Plapp, J. Biol. Chem. 1991, 266, 13296 -13302; b) S. Al-Karadaghi, E. S. Cedergren-Zeppezauer, S. Hovmoller, K. Petratos, H. Terry, K. S. Wilson, Acta Crystallogr. Sect. D 1994, 50, 793 - 807) or the alcohol dehydrogenase from *Geobacillus stearothermophilus* (ADH-hT).( a) R. Cannio,M. Rossi, S. Bartolucci, Eur. J. Biochem. 1994, 222, 345 - 352; b) X. Zhang, T. C. Bruice, Biochemistry 2007, 46, 837-843.)

The oxidation of a racemic mixture of secondary alcohols is performed by two stereocomplementary alcohol dehydrogenases. A suitable Prelog (V. Prelog, Pure Appl. Chem. 1964, 9, 119 - 130) NADH-dependent alcohol dehydrogenase is the alcohol dehydrogenase 'A' from *Rhodococcus ruber* (ADH-A) (B. Kosjek, W. Stampfer, M. Pogorevc, W. Goessler,K. Faber, W. Kroutil, Biotechnol. Bioeng. 2004, 86, 56 - 62) Various other Prelog ADHs are in principle suitable.

Anti-Prelog NADH-dependent alcohol dehydrogenases are rare in nature. Enzymes from *Leifsonia sp.* and *Lactobacillus brevis* have been described in literature. (a) K. Inoue, Y. Makino, T. Dairi, N Itoh, Biosci. Biotechnol. Biochem. 2006, 70, 418 - 426. b) S. Leuchs, L. Greiner, Chem.Biochem.Eng.Q. 2011, 25 267-281) However, in this invention, an engineered ADH from *Lactobacillus brevis* (LBv-ADH) has been employed as an anti-Prelog ADH (N. H. Schlieben, K. Niefind, J. Muller, B. Riebel, W. Hummel, D. Schomburg, J.Mol.Biol. 2005, 349 801-813).

**1.1.1 Horse liver alcohol dehydrogenase (HL-ADH) (SEQ ID NO:8)**

The gene for the HL-ADH was cloned in a pET-28a vector. Cloning was performed in a way that the enzyme was going to be overexpressed as N-terminal $His_6$-tagged enzyme. The plasmid was transformed in *E. coli* BL21 according to the standard protocol from the supplier. Colonies were cultivated on an agar plate LB/Kanamycin (10 $g*L^{-1}$ tryptone, 5 $g*L^{-1}$ yeast extract, 10 $g*L^{-1}$ NaCl, 20 $g*L^{-1}$ agar, 50 $mg*L^1$kanamycin). A single colony of *E. coli* BL21/HL-ADH was taken from the agar plate and it was inoculated in LB/Kan (*v.s.* w/o agar)and grown overnight at 37 °C, 170 rpm, for 16 h. Then, 4 large cultures (4 × 800 mL) constituted by LB/Kan media (50 mg $L^{-1}$), supplemented with $Zn^{2+}$ (100 mg $L^{-1}$ of $ZnCl_2$), were inoculated with the overnight culture (15 mL each). The $OD_{600}$ was checked after 2 h and it was found to be slightly more than 0.7. Then, cultures were induced with IPTG (final concentration 0.5 mM). Cultures were shaken at 170 rpm, at 20 °C, for 24 h. Cultures were harvested, centrifuged and the pellets were washed with an aqueous solution of NaCl (5% w $w^{-1}$). Pellets were frozen and stored at -20 °C.

**1.1.2 Alcohol dehydrogenase 'A' from *Rhodococcus ruber* (ADH-A) (SEQ ID NO:9)**

The gene for the ADH-'A' from *Rhodococcus ruber was* cloned in a pET-21 a vector. Cloning was performed in a way that the enzyme was going to be overexpressed as C-terminal $His_6$-tagged enzyme. The plasmid was transformed in *E. coli* Tuner (DE3) according to the standard protocol from the supplier. Colonies were cultivated on an agar plate LB/Ampicillin (100 mg $L^{-1}$). A single colony of *E. coli* BL21/ADH-'A' was taken from the agar plate and it was inoculated in LB/Amp (10 $g*L^{-1}$ tryptone, 5 $g*L^{-1}$ yeast extract, 10 $g*L^{-1}$ NaCl, 20 $g*L^{-1}$ agar, 100 $mg*L^{-1}$ ampicillin) and grown overnight at 37 °C, 170 rpm, for 16 h. Then, 6 large cultures (4 × 500 mL) constituted by LB/Amp (100 mg $L^{-1}$), supplemented with $Zn^{2+}$ (100 mg $L^{-1}$ of $ZnCl_2$), were inoculated with the overnight culture (10 mL each). The cultures were grown for 24 h, at 30 °C and 170 rpm. Then, cultures were induced with IPTG (final concentration 2 mM) and further shaken at 170 rpm, at 20 °C, for 24 h. Cultures were harvested, centrifuged and the pellets were washed with an aqueous solution of NaCl (5% w $w^{-1}$). Pellets were frozen and stored at -20 °C.

**1.1.3 Alcohol dehydrogenase from *Lactobacillus brevis* (LBv-ADH) (SEQ ID NO:6)**

The preparation was done as disclosed in Schlieben, N.H. et al., Journal of Molecular Biology, 2005, 349, (4), 801.

**1.1.4 Alcohol dehydrogenase from *Aromatoleum aromaticum* (AA-ADH) (SEQ ID NO:5)**

The preparation was done as disclosed inH. W. Höffken, Duong Minh, T. Friedrich, M. Breuer, B. Hauer, R. Reinhardt, R. Rabus, J. Heider, Biochemistry 2006, 45 82-93...

**1.2 Enzymes of the reductive step (Amination)**

The reductive amination step of the exemplified method is performed by amine dehydrogenases (AmDH) which are strictly NADH-dependent. These enzymes convert ketones (i.p. possessing a lower alkyl substituent) as well as aldehydes.

In particular, two amine dehydrogenases have been employed. The first enzyme is a K78S, N277L variant originated from the wild-type phenylalanine dehydrogenase from *Bacillus badius* (Ph-AmDH). (EC 1.4.1.20, UniProt-ID:

Q59224). The second enzyme is a K68S, E114V, N262L, V291C variant originated from the wild-type leucine dehydrogenase from *Geobacillus stearothermophilus* (L-AmDH) (EC 1.4.1.9, GenBank-ID:M22977).

Both genes have been custom-synthesized chemically (cf. Carlson, R. Nat. Biotechnol., 2009, 27, 1091) and cloned into the respective expression vectors.

### 1.2.1 Amine dehydrogenase variant originated from the phenylalanine dehydrogenase from *Bacillus badius* (Ph-AmDH) (SEQ ID NO: 2)

The gene for the Ph-AmDH variant originated from the phenylalanine dehydrogenase from *Bacillus badius* and was cloned in a pET-28b vector. Cloning was performed in a way that the enzyme was going to be overexpressed as N-terminal $His_6$-tagged enzyme. The plasmid was transformed in *E. coli* BL21 (DE3) according to the standard protocol from the supplier. Colonies were cultivated on an agar plate LB/Kanamycin (50 mg $L^{-1}$). A single colony of *E. coli* BL21/Ph-AmDH was taken from the agar plate and it was inoculated in LB/Kan (50 mg $L^{-1}$) and grown overnight at 37 °C, 170 rpm, for 16 h. Then, 4 large cultures (4 × 800 mL) constituted by LB/Kan (50 mg $L^{-1}$) were inoculated with the overnight culture (15 mL each). The $OD_{600}$ was checked after 2 h and it was found to be around 0.7. Then, cultures were induced with IPTG (final concentration 0.5 mM) and further shaken at 170 rpm, at 20 °C, for 24 h. Cultures were harvested, centrifuged and the pellets were washed with an aqueous solution of NaCl (5% w $w^{-1}$). Pellets were frozen and stored at -20 °C. The total amount of wet cells was 15.2 g.

Two $Ni^{2+}$ columns (5 mL × 2) were washed with water (50 mL) and conditioned with lysis buffer (10 mM imidazole, pH 8, 50 mM $KH_2PO_4$, 300 mM NaCl, 50 mL) at a flow of 2.4 mL $min^{-1}$. The wet cells containing the AmDH variant (ca. 9.2 g) were suspended in the lysis buffer (40 mL, 10 mM imidazole buffer, pH 8, 50 mM $KH_2PO_4$, 300 mM NaCl).

The whole cells were initially disrupted with lysozyme (1 mg $mL^{-1}$) by shaking at 150 rpm, at 20 °C for 30 - 40 minutes. The mixture was diluted with the lysis buffer up to 80 mL. Then, the disruption was completed by sonication (5 min, amplitude 45%, pulse on 20 s, pulse off 40 s). The suspension was centrifuged (20000 rpm, 1 h, 4 °C) and the supernatant was filtrated first through a 0.45 μm filter and then through 0.20 μm filter. The solution was the loaded into the previously conditioned columns (flow 2 mL $min^{-1}$). The columns were washed with the washing buffer (imidazole 25 mM, pH 8, 50 mM $KH_2PO_4$, NaCl 300 mM, 75 mL) at a flow of 2 mL $min^{-1}$. The column was then eluted with the elution buffer 1 (5 mL × 14 fractions, imidazole 200 mM, pH 8, 50 mM $KH_2PO_4$, NaCl 300 mM). Then the concentration of imidazole was increased using an elution buffer 2 (7-8 mL × 5 fractions, imidazole 350 mM, pH 8, 50 mM $KH_2PO_4$, NaCl 300 mM). A Bradford assay as well as SDS-gel page showed that the AmDH eluted from fraction 2 to fraction 18. SDS-gel page showed that AmDH was obtain with elevate purity (> 99%).

The purified enzyme solution was dialyzed overnight against potassium phosphate buffer (8 L, pH 8, 50 mM). The enzyme was concentrated from 40 mL up to 9 mL. The enzyme concentration was 53 mg $mL^{-1}$. The final amount of AmDH obtained was therefore 477 mg, obtained from 9.2 g of wet cells, equal to 1.9 L of culture (without optimization).

### 1.2.2 Amine dehydrogenase variant originated from the leucine dehydrogenase from *Geobacillus stearothermophilus* (L-AmDH). (SEQ ID NO:4)

The preparation was done by conventional methods, as for example described above for Alcohol dehydrogenase 'A' from Rhodococcus ruber (ADH-A)

### Example 2: Redox self-sufficient biocatalytic cascade for amination of alcohols

The cascade depicted in Scheme 4 has been performed:

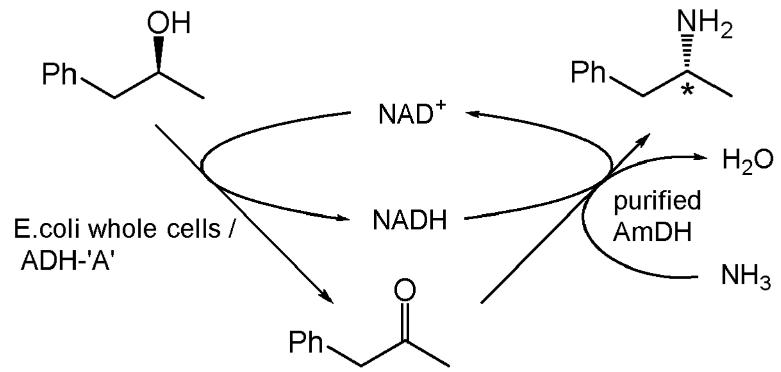


**Scheme 4. Proof of principle for the redox self-sufficient amination of alcohols combining an ADH with an AmDH.**

A preliminary experiment of combining the Prelog alcohol dehydrogenase ADH-'A' with the amine dehydrogenase Ph-AmDH was performed. The ADH-'A' was used in the form of *E. coli* whole cells (ca. 10 mg whole cells) overexpressing the catalyst without any further purification. The AmDH was used as a purified enzyme (50 or 100 µL, 53 mg $mL^{-1}$). The AmDH was purified as described previously. (*S*)-phenyl 2-propanol (20 mM) was used as the test substrate. The reaction was run in ammonia / ammonium chloride buffer (1 mL, 240 mM, pH 9.6), prepared as previously described. Catalytic amount of cofactor $NAD^+$ was used (1 mM). The reaction was shaken at 30 °C, for 1 day, in an orbital shaker at 170 rpm. Work-up was performed adding KOH (100 µL, 10N) and then extracting with EtOAc (2 × 500 µL). The amine product (*R*)-phenyl 2-propylamine was obtained with 25% conversion perfect chemoselectivity and in optically pure form (ee >99%). Hence, we demonstrated the feasibility of the two enzymes cascade.

**Example 3: pH study for the amination using the Ph-AmDH N-term $His_6$-tagged**

The enzyme Ph-AmDH (AmDH variant from the WT phenylalanine dehydrogenase from *Bacillus badius*) was investigated for the amination at different pH and using various buffer systems (scheme 3):

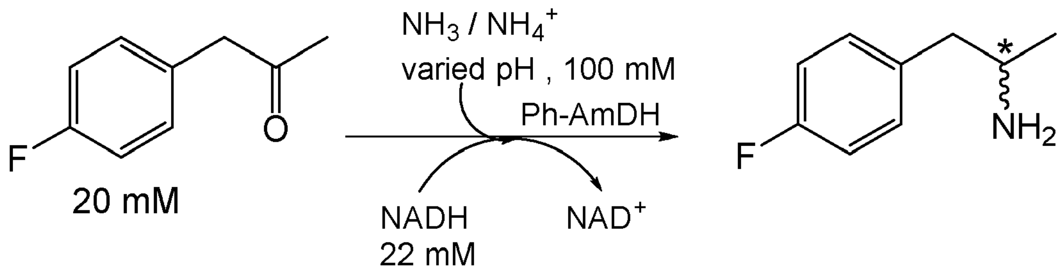


**Scheme 3. Reductive amination using Ph-AmDH**

The buffers were constituted by ammonia and ammonium species. The counter-anions for the ammonium cations were derived from chloride, sulfate, acetate, phosphate, borate, citrate, oxalate or formate.

*para*-Fluoro-phenyl-acetone was used as the test substrate (20 mM). In this set of experiments, NADH was used in stoichiometric amount with 10% excess (1.1 eq, 22 mM).

The buffers were prepared starting from a solution of ammonia in distilled water (100 mM of ammonia concentration). The initial pH was ca. 11.5. Then, the various buffer systems with different ion species and at different pH were prepared by taking an aliquot of this ammonia solution and adjusting the pH to different values through the addition of subsequent aliquots of a given acid (hydrochloric acid, sulfuric acid, acetic acid, phosphoric acid, boric acid, citric acid, oxalic acid, formic acid). The pH ranges investigated obviously depended on the nature of acid employed in combination with the ammonia. For instance, the ammonium chloride buffer system exists between pH 8.2 and the initial pH ca. 11.5. Hence the biocatalytic reductive amination was studied in this range of pH in the ammonium chloride buffer. Other weaker acids allow for studying also neutral as well as moderate acid pH.

The results are summarized in figure 1. Independently from the type of buffer of choice, the highest conversion

was always observed at pH between 8 and 9. Among all the buffer systems tested, ammonium chloride at pH 8.2 - 8.7 gave the best result in terms of conversion. For this reason, the ammonium chloride buffer at pH 8.7 was chosen at the beginning as the buffer for further experiments.

The enantiomeric excess for the bio-amination was also measured. The ee% value of the amine product was >99% (R)

Experimental Details:

Each sample was prepared by mixing the specific ammonia / ammonium ion buffer (450 μL) with the enzyme solution (50 μL, 20 U, stored in phosphate buffer pH 8, 50 mM). The pH of the ammonia / ammonium ion buffer did not vary upon addition of the enzyme solution, due to the high concentration of the ammonia / ammonium ion in the buffer. NADH was used in stoichiometric amount (1.1 eq, 22 mM, 15.6 mg $mL^{-1}$) and pre-dissolved in the ammonia buffer. Substrate para-fluoro-phenylacetone was finally added (20 mM, 1.34 μL). Reactions were shaken at 30 °C, in an orbital shaker at 150 rpm, for 38 h. The reactions were stopped by adding KOH (100 μL, 10 N), samples were extracted with EtOAc (2 × 300 μL) and analysed by GC-FID.

The enantiomeric excess of the amine was analyzed by GC on a chiral phase after derivatization to the corresponding acetoamides. Derivatization was performed by adding 4-(N,N-dimethylamino)pyridine (3 mg) dissolved in acetic anhydride (50 mL). After washing with water and drying ($MgSO_4$) the ee value of the derivatized compound was measured.

**Example 4: Reductive amination using the Ph-AmDH with catalytic NADH in ammonia / ammonium chloride buffers at various concentrations**

The reductive amination of the test substrate para-fluroro-phenylacetone (20 mM) was carried out in ammonium chloride buffer (pH 8.7) at various ammonium / ammonia concentrations (here the concentration is referred to the sum of free ammonia and ammonium cation species present in solution). In this set of experiments, the cofactor NADH was used in catalytic amount (1 mM) and it was recycled using glucose dehydrogenase (GDH) and glucose (scheme 4):

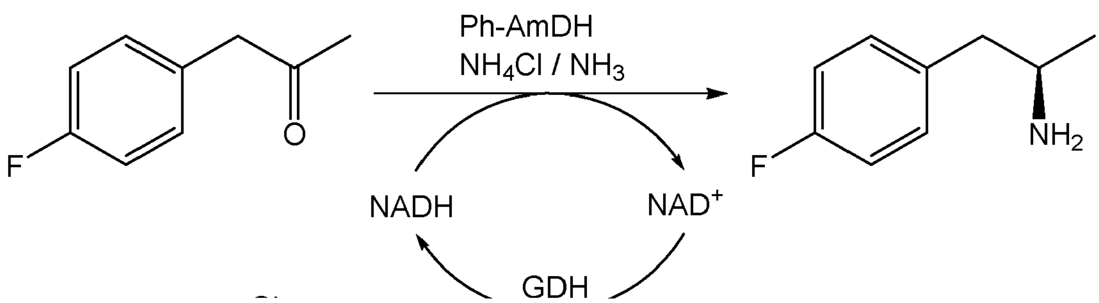


**Scheme 4. Biocatalytic reductive amination using Ph-AmDH and catalytic NADH.**

The reactions were carried out in the following ammonium chloride buffers at pH 8.7: 100 mM - 200 mM - 381 mM - 566 mM - 727 mM - 893 mM - 1293 mM - 1695 mM. The reactions were carried out at 30 °C for 16 h. Results are summarized in table 1:

**Table 1**

| Sample | Ammonium / ammonia concentration [mM] | Conversion [%] |
|---|---|---|
| 1 | 100 | 19.25 |
| 2 | 200 | 75.79 |
| 3 | 381 | 91.44 |
| 4 | 566 | 97.28 |
| 5 | 727 | 99.61 |
| 6 | 893 | 99.66 |
| 7 | 1293 | 99.92 |

(continued)

| Sample | Ammonium / ammonia concentration [mM] | Conversion [%] |
|---|---|---|
| 8 | 1695 | 99.95 |

The biocatalytic reductive amination of *para*-fluoro-phenylacetone (20 mM) led to quantitative conversion (>99.5) when ca. 700 mM of ammonia / ammonium was applied. The cofactor NADH was used in catalytic amount (1 mM) and recycled with GDH / glucose. The ee of the amine product was >99% (R)

Experimental Details:

Each sample was prepared using the specific ammonia / ammonium chloride buffer (450 μL) containing the catalytic NADH (1 mM, 0.76 mg $mL^{-1}$), GDH (Codexis, 5 mg, crude lyophilised extract) and glucose (3 eq., 60 mM, 10.8 mg $mL^{-1}$). Then, the enzyme solution was added (50 μL, 20 U, stored in phosphate buffer pH 8, 50 mM). The pH of the ammonia / ammonium chloride buffer did not vary significantly upon addition of the enzyme solution, due to the high concentration of the ammonium in the buffer. Substrate para-fluoro-phenylacetone was finally added (20 mM, 1.34 μL). Reactions were shaken at 30 °C, in an orbital shaker at 150 rpm, for 16 h. The reactions were stopped by adding KOH (100 μL, 10 N). Samples were extracted with EtOAc (2 × 300 μL) and analysed by GC-FID.

Enantiomeric excess was measured as previously described.

**Example 5: Time study for the reductive amination using ammonium chloride / ammonia buffer (pH 8.7, 727 mM)**

In the previous set of experiments, it was shown that the biocatalytic reductive amination of *para*-fluoro-phenylacetone (20 mM) afforded quantitative conversion within 16 h when a 35-fold excess of ammonia / ammonium species was applied. In this further experiment, we monitored the conversion of the substrate into the amine product during the time under the same reaction conditions (pH 8.7, 727 mM ammonia / ammonium species, 30 °C). Catalytic NADH (1 mM) was recycled with GDH and glucose as previously described.

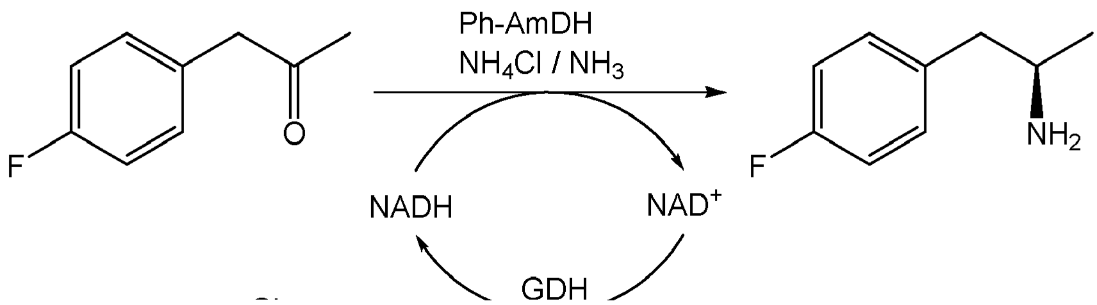


The results are depicted in Figure 2. Under these reaction conditions, quantitative conversion was achieved after 12 h. The ee of the amine product was >99% (R)

Experimental Details:

Each sample was prepared using the ammonia / ammonium chloride buffer (450 μL) containing catalytic NADH (1 mM, 0.76 mg $mL^{-1}$), GDH (Codexis, 5 mg, crude lyophilised extract) and glucose (3 eq., 60 mM, 10.8 mg $mL^{-1}$). Then, the enzyme solution was added (50 μL, 20 U, stored in phosphate buffer pH 8, 50 mM). After addition of the enzyme solution, the final concentration of ammonia plus ammonium cations was 727 mM. The pH of the ammonia / ammonium chloride buffer does not vary significantly upon addition of the enzyme solution, due to the high concentration of the ammonium in the buffer. Substrate para-fluoro-phenylacetone was finally added (20 mM, 1.34 μL). Reactions were shaken at 30 °C, in an orbital shaker at 150 rpm. The reactions were stopped by adding KOH (100 μL, 10 N). Samples were extracted with EtOAc (2 × 300 μL) and analysed by GC-FID.

Enantiomeric excess was measured as previously described.

**Example 6: Substrate scope of the Ph-AmDH**

The substrate scope of the Ph-AmDH for the conversion of a panel of ketone substrates has never been never

determined in the prior art. Only initial reaction rates for few substrates have been determined through a spectrophotometric assay. Hence the biocatalytic reductive amination catalysed by Ph-AmDH was investigated towards a panel of substrates including aryl- as well as alkyl-ketones (Figure 3):

Substrate concentration was 20 mM. Ammonia / ammonium chloride buffer (pH 8.7, 727 mM) was used. Catalytic NADH (1 mM) was recycled as previously mentioned using GDH and glucose. Reactions were performed at 30 °C for 16 h. Results are reported in table 2.

**Table 2**

| Substrate | Conversion |
|---|---|
| 1 | >99 |
| 2 | 22 |
| 3 | 15 |
| 4 | 88 |
| 5 | 5 |
| 6 | 10 |
| 7 | 71 |
| 8 | 68 |
| 9 | n. c. |
| 10 | 26 |
| 11 | 32 |
| 12 | 3 |
| 13 | 2 |
| 14 | 10 |
| 15 | 96 |
| n.c. = no conversion | |

All the substrates tested were converted with the exception of acetophenone.

The ee was measured for the conversion of substrate 1. The ee was >99% (R).

Experimental Details:

Each sample was prepared using the ammonia / ammonium chloride buffer (450 μL) containing catalytic NADH (1 mM, 0.76 mg $mL^{-1}$), GDH (Codexis, 5 mg, crude lyophilised extract) and glucose (3 eq., 60 mM, 10.8 mg $mL^{-1}$). Then, the enzyme solution was added (50 μL, 20 U, stored in phosphate buffer pH 8, 50 mM). After addition of the enzyme solution, the final concentration of ammonia plus ammonium cations was 727 mM. The pH of the ammonia / ammonium chloride buffer did not vary significantly upon addition of the enzyme solution, due to the high concentration of the ammonium in the buffer. Substrate was finally added (20 mM). Reactions were shaken at 30 °C, in an orbital shaker at 150 rpm, for 16 h. The reactions were stopped by adding KOH (100 μL, 10 N). Samples were extracted with EtOAc (2 × 300 μL) and analysed by GC-FID.

The ee was measured as previously described.

**Example 7: Hydrogen-borrowing bio-amination of alcohols with stereoinversion**

In this set of experiments the amination of (S)-phenyl-2-propanol (20 mM) was carried out by combining a Prelog alcohol dehydrogenase from *Aromatoleum aromaticum* (AA-ADH) with the Ph-AmDH. Since the Ph-AmDH is (*R*)-selective, the overall bio-amination proceeded with inversion of configuration. The enzymes were used in their purified form. The bio-amination was carried out in ammonia / ammonium chloride buffer at pH 8.7.

The initial experiments were performed using (*S*)-phenyl-2-propanol (20 mM) and catalytic $NAD^+$ at 1 mM concentration (5 mol%). In this first set of experiments, the concentration of ammonia / ammonium chloride was varied from 100 mM to 4680 mM in order to investigate the impact on the conversion after 24 h reaction time, at 30 °C. Ammonia

/ ammonium chloride buffers were used at the following concentrations: 100 mM - 200 mM - 400 mM - 1000 mM - 1500 mM - 2000 mM - 2500 mM - 3000 mM - 3500 mM - 4000 mM - 4680 mM. The results are reported in table 3.

**Table 3**

| $NH_4^+/NH_3$ [mM] | Amine | Alcohol | Ketone |
|---|---|---|---|
| 100 | 4.86 | 91.61 | 3.44 |
| 200 | 11.55 | 83.74 | 4.71 |
| 400 | 31.50 | 62.19 | 6.31 |
| 1000 | 59.05 | 36.01 | 4.94 |
| 1500 | 75.20 | 20.42 | 4.38 |
| 2000 | 87.14 | 8.53 | 4.33 |
| 2500 | 83.52 | 12.31 | 4.17 |
| 3000 | 84.84 | 10.83 | 4.33 |
| 3500 | 74.72 | 19.89 | 5.39 |
| 4000 | 80.56 | 14.54 | 4.90 |
| 4680 | 59.75 | 35.08 | 5.17 |

In this first set of experiments, the highest conversion observed after 24 h reaction time was 87%. The ee of the amine product was > 99% (*R*). This was achieved using 2 M of ammonia / ammonium chloride. The substrate was used in 20 mM concentration. Therefore 2 M ammonia / ammonium chloride buffer was used for further experiments.

In the second set of experiments, the conversion for the amination of (*S*)-phenyl-2-propanol (20 mM) - combining the Prelog alcohol dehydrogenase from *Aromatoleum aromaticum* (AA-ADH) with the Ph-AmDH - was monitored during the time. Different samples were prepared and quenched at different times: 1 h - 3 h - 6 h - 12 h - 18 h - 24 h - 48 h - End (144 h). The concentration of the substrate was kept at 20 mM, while the concentration of the cofactor $NAD^+$ was varied: 1 mM - 0.5 mM - 0.2 mM - 0.1 mM. The results for these experiments are reported in table 4 and in Figure 4, 5, 6 and 7.

The ee of the final product was >99% (R).

**Table 4**

| Time [h] | Conversion [%] | | | | | | | | | | | |
|---|---|---|---|---|---|---|---|---|---|---|---|---|
| | $NAD^+$ [1 mM] | | | $NAD^+$ [0.5 mM] | | | $NAD^+$ [0.2 mM] | | | $NAD^+$ [0.1 mM] | | |
| | Am | Alc | Ket | Am | Alc | Ket | Am | Alc | Ket | Am | Alc | Ket |
| **1** | 4.02 | 89.91 | 6.07 | 2.07 | 94.79 | 3.14 | 1.30 | 97.07 | 1.63 | 0.57 | 98.51 | 0.92 |
| **3** | 11.77 | 81.20 | 7.03 | 8.44 | 86.89 | 4.67 | 5.07 | 91.85 | 3.08 | 1.74 | 95.99 | 2.27 |
| **6** | 25.78 | 68.21 | 6.01 | 9.91 | 87.01 | 3.08 | 7.54 | 90.76 | 1.70 | 2.87 | 96.12 | 1.01 |
| **12** | 47.80 | 46.56 | 5.64 | 37.50 | 59.46 | 3.04 | 13.87 | 84.77 | 1.36 | 5.95 | 93.27 | 0.78 |
| **18** | 58.55 | 36.28 | 5.17 | 40.02 | 57.01 | 2.97 | 22.03 | 76.51 | 1.46 | 8.58 | 90.55 | 0.87 |
| **24** | n.d. | n.d. | n.d. | n.d. | n.d. | n.d. | 35.15 | 63.55 | 1.30 | 15.56 | 83.44 | 1.00 |
| **48** | 91.10 | 5.74 | 3.16 | 87.00 | 10.51 | 2.49 | 40.07 | 58.19 | 1.74 | n.d. | n.d. | n.d. |
| **End** | 93.15 | 4.91 | 1.94 | 92.61 | 5.96 | 1.43 | 75.92 | 22.86 | 1.22 | 34.35 | 64.86 | 0.79 |
| Am = (*R*)-phenyl-2-propylamine<br>Alc = (*S*)-phenyl-2-propanol<br>Ket = phenyl-acetone | | | | | | | | | | | | |

Experimental Details:

The AA-ADH was overexpressed in *E. coli* and supplied by BASF as frozen crude cell extract. An aliquot of the crude cell extract was defrosted and centrifuged. Cell debris was removed and the supernatant was passed first through a desalting column. Then, the enzyme solution was purified via ion exchange chromatography (IEC) and size exclusion chromatography (SEC). The purified enzyme solution was loaded into a PD10 column and eluted with the storage buffer (Tris-HCl pH 8, 50 mM containing $ZnSO_4$ 0.1 mM). The enzyme solution was concentrated, frozen in liquid nitrogen and stored at -80 °C.

The Ph-AmDH carrying an N-terminal $His_6$ tag was overexpressed in *E. coli.* After cell disruption, the lysate was centrifuged and cell debris was removed. The supernatant was filtered and the enzyme solution was purified via $Ni^{2+}$ affinity chromatography. The purified enzyme solution was dialysed in phosphate buffer (pH 8, 50 mM). The N-terminal $His_6$ tag of the Ph-AmDH was then cleaved using biotinylated thrombin. The residual thrombin was removed by addition of streptavidin agarose beads followed by centrifugation and filtration. The Ph-AmDH was separated from the cleaved $His_6$ tag using again $Ni^{2+}$ affinity chromatography. The final enzyme solution was dialysed overnight in phosphate buffer (pH 8, 50 mM), concentrated, frozen in liquid nitrogen and stored at -80 °C.

*Representative biocatalytic amination:* Each sample was prepared using the ammonia / ammonium chloride buffer (430 μL) containing catalytic $NAD^+$ (final concentration 1 mM, 0.76 mg $mL^{-1}$). The AA-ADH enzyme solution (20 μL, 47 mg $mL^{-1}$, in Tris-HCl Buffer pH 8, 50 mM) and the Ph-AmDH solution (50 μL, 20 U, stored in phosphate buffer pH 8, 50 mM) were added. (The pH of the ammonium chloride buffer did not vary significantly upon addition of the enzyme solutions, due to the high concentration of the ammonia / ammonium ion in the buffer. The reported concentration of the ammonia / ammonium ion in solution takes into account the effect of the dilution due to the addition of the enzyme solutions). The substrate was finally added (20 mM, 1.34 μL). Reactions were shaken at 30 °C, in an orbital shaker at 150 rpm, for 24 h. The reactions were stopped by adding KOH (100 μL, 10 N). Samples were extracted with EtOAc (2 × 300 μL) and analysed by GC-FID.

The same protocol was applied for the time studies. The only difference was the concentration of the cofactor $NAD^+$ that was varied (1 mM, 0.5 mM, 0.2 mM, 0.1 mM).

The ee was determined as previously described.

**Example 8: Hydrogen-borrowing bio-amination of alcohols with retention of configuration**

In this set of experiments the amination of (R)-phenyl-2-propanol (20 mM) was carried out combining an anti-Prelog alcohol dehydrogenase variant originated from *Lactobacillus brevis* (LBv-ADH) with the Ph-AmDH. Since the Ph-AmDH is (*R*)-selective, the overall bio-amination proceeded with retention of configuration. The enzymes were used in their purified form. The initial attempt for this bio-amination was carried out in ammonia / ammonium chloride buffer at pH 8.7 as previously described.

The initial experiments were performed using (*R*)-phenyl-2-propanol (20 mM) and catalytic $NAD^+$ at 1 mM concentration (5 mol%). In this first set of experiments, the concentration of ammonia / ammonium chloride was varied from 200 mM to 2500 mM in order to investigate the impact on the conversion after 24 h reaction time, at 30 °C.

Ammonia / ammonium chloride buffers were used at the following concentrations: 200 mM - 500 mM - 1000 mM - 1500 mM - 2000 mM - 2500 mM. The results are reported in table 5.

**Table 5**

| $NH_4^+$ / $NH_3$ [mM] | Amine | Alcohol | Ketone |
|---|---|---|---|
| 200 | 1.37 | 96.40 | 2.23 |
| 500 | 1.31 | 97.18 | 1.51 |
| 1000 | 0.59 | 98.45 | 0.96 |
| 1500 | 0.16 | 99.19 | 0.65 |
| 2000 | n.d. | 99.44 | 0.56 |
| 2500 | n.d. | 99.42 | 0.58 |

In this first set of experiments, the highest conversion observed after 24 h reaction time was only 1.37%. In fact, enzyme precipitation (probably the LBv-ADH) occurred under the employed reaction conditions.

Due to the lower stability of the LBv-ADH at pH > 8.5 in presence of ammonium species, we decided to perform this cascade at lower pHs. However, the theoretical lower pH limit for the ammonium chloride buffer is about 8.2. Hence,

the buffer capacity of the ammonium chloride buffer is already extremely low at pH 8.5. Therefore, we decided to change the type of buffer for this cascade. Among the buffer systems tested for the reductive amination with the Ph-AmDH (as reported above), the best were formate, oxalate, acetate and citrate. We selected ammonium formate as the buffer system since it shows the highest buffer capacity at pH around 7. Furthermore, oxalate, acetate and citrate can act as chelators. This must be avoided due to the fact that $Zn^{2+}$ is the essential catalytic ion for the alcohol dehydrogenases that are employed in the present invention. Furthermore, the LBv-ADH possesses a coordinated $Mg^{2+}$ that is essential for its stability (i.e. structural $Mg^{2+}$). Furthermore, exogenous $Mg^{2+}$ is also added in the reaction buffer. This was shown to increase stability of the wild-type LB-ADH during purification.

$HCOONH_4$ / $NH_3$ buffer containing $Mg^{2+}$ (as $MgCl_2$ hexa-hydrate in 1 mM concentration) was employed in this set of experiments. The experiments were performed using (*R*)-phenyl-2-propanol (20 mM) and catalytic $NAD^+$ at 1 mM concentration (5 mol%). The pH was varied: 7 - 7.5 - 8 - 8.5. The concentration of the ammonia / ammonium ion was varied: 0.5 M - 1 M - 2 M. Reaction was run at 30 °C, 180 rpm, on orbital shaker, for 24 h. The results are reported in table 6.

The ee of the final product was >99% (R).

**Table 6**

| Sample | pH | $NH_3/NH_4^+$ concentration [M] | Conversion [%] | | |
|---|---|---|---|---|---|
| | | | Amine | Alcohol | Ketone |
| 1 | 7 | 0.5 | 4.04 | 94.20 | 1.76 |
| 2 | 7 | 1 | 8.14 | 90.03 | 1.83 |
| 3 | 7 | 2 | 21.11 | 76.75 | 2.14 |
| 4 | 7.5 | 0.5 | 5.17 | 92.95 | 1.88 |
| 5 | 7.5 | 1 | 19.26 | 78.17 | 2.57 |
| 6 | 7.5 | 2 | 73.64 | 22.52 | 3.84 |
| 7 | 8 | 0.5 | 4.50 | 93.39 | 2.11 |
| 8 | 8 | 1 | 14.04 | 83.09 | 2.86 |
| 9 | 8 | 2 | 93.80 | 2.08 | 4.12 |
| 10 | 8.5 | 0.5 | 2.56 | 94.93 | 2.51 |
| 11 | 8.5 | 1 | 7.90 | 89.61 | 2.48 |
| 12 | 8.5 | 2 | 73.85 | 22.06 | 4.09 |

In this set of experiments, the highest conversion observed after 24 h reaction time was 93.80%. This was achieved using 2 M of ammonia / ammonium formate buffer at pH 8. The substrate was used in 20 mM concentration.

Experimental Details:

The LBv-ADH was overexpressed in *E. coli* and supplied by BASF as frozen crude cell extract. An aliquot of the crude cell extract was defrosted and centrifuged. Cell debris was removed and the supernatant was passed first through a desalting column. Then, the enzyme solution was purified via ion exchange chromatography (IEC) and size exclusion chromatography (SEC). The purified enzyme solution was loaded into a PD10 column and eluted with the storage buffer (Tris-HCl pH 8, 50 mM containing $MgCl_2$ 1 mM and $NAD^+$ 0.1 mM). The enzyme solution was concentrated, frozen in liquid nitrogen and stored at -80 °C.

The Ph-AmDH was purified as previously described.

*Representative biocatalytic amination:* Each sample was prepared using the ammonia / ammonium formate buffer (425 μL) containing catalytic $NAD^+$ (final concentration 1 mM, 0.76 mg $mL^{-1}$). The LBv-ADH enzyme solution (25 μL, 38 mg $mL^{-1}$, in Tris-HCl Buffer pH 8, 50 mM) and the Ph-AmDH solution (50 μL, 20 U, stored in phosphate buffer pH 8, 50 mM) were added. (The pH of the ammonium formate buffer did not vary significantly upon addition of the enzyme solutions, due to the high concentration of the ammonia / ammonium ion in the buffer. The reported concentration of the ammonia / ammonium ion in solution takes into account the effect of the dilution due to the addition of the enzyme solutions). The substrate was finally added (20 mM, 1.34 μL). Reactions were shaken at 30 °C, in an orbital shaker at 150 rpm, for 24 h. The reactions were stopped by adding KOH (100 μL, 10 N). Samples were extracted with EtOAc (2 × 300 μL) and analysed by GC-FID.

The ee was determined as previously described.

**Table 7**: List of SEQ ID NOs

| SEQ ID NO: | Short name | Description/Origin | Type |
|---|---|---|---|
| 1 | Ph-DH | *Bacillus badius* (Wildtype-Sequence) | AS |
| 2 | Ph-AmDH | Mutant of Ph-DH with amine dehydrogenase activity | AS |
| 3 | L-DH | *Geobacillus stearothermophilus* (Wildype-Sequence) | AS |
| 4 | L-AmDH | Mutant of L-DHwith amine dehydrogenase activity | AS |
| 5 | AA-ADH | *Aromatoleum aromaticum* (Wildtype Sequence) | AS |
| 6 | LBv-ADH | *Lactobacillus brevis* (Mutant Sequence) | AS |
| 7 | GDH | *Bacillus subtilis* | AS |
| 8 | HL-ADH | *Equus ferus cabalus* | AS |
| 9 | ADH-A | *Rhodococcus ruber* | AS |
| 10 | ADH-hT | *Geobacillus stearothermophilus* | AS |
| AS = Amino acid sequence<br>NS = Nucleotide sequence | | | |

Documents as cited herein are incorporated by reference

## Claims

**1.** A method of preparing an amine, which method comprises:

a) the enzymatic oxidation of the corresponding alcohol of said amine in the presence of the cofactor $NAD(P)^+$ and of an alcohol dehydrogenase (ADH) (E.C.1.1.1.x) to form the corresponding carbonyl compound of said alcohol, while the cofactor $NAD(P)^+$ is reduced to form NAD(P)H; and
b) the enzymatic reduction (reductive amination) of said carbonyl compound in the presence of

i)) ammonia or an ammonia source, and
ii) an amine dehydrogenase (AmDH)

to form the desired amine,
while the cofactor $NAD(P)^+$ is regenerated from NAD(P)H; and optionally
c) isolating said amine.

**2.** The method of claim 1, wherein steps a) and b) are performed simultaneously.

**3.** The method of one of the preceding claims, wherein said AmDH is obtained by protein engineering of an amino acid dehydrogenase enzyme, in particular oxi-doreductases acting on the $CH-NH_2$ group of donor molecules (substrates) with NAD or NADP as acceptor, (E.C. 1.4.1.).

**4.** The method of one of the preceding claims, wherein said AmDH is a mutant of an amino acid dehydrogenase enzyme, which mutant has the ability to convert, in the presence of ammonia or an ammonia source and NAD(P)H, an carbonyl compound to the corresponding amine.

**5.** The method of claim 4 or 5, wherein said AmDH is a mutant of a phenylalanine dehydrogenase (Ph-AmDH) (E.C. 1.4.1.20) or a mutant of a leucine dehydrogenase (L-AmDH) (E.C. 1.4.1.9).

**6.** The method of claim 5, wherein said AmDH is selected from a

a) a mutant of a phenyl alanine dehydrogenase comprises an amino acid sequence of SEQ ID NO: 1, wherein said mutant comprises the double mutation K78S,N277L, and wherein said mutant has a sequence identity of

at least 80 % to SEQ ID NO: 1, and
b) a mutant of a leucine dehydrogenase comprises an amino acid sequence of SEQ ID NO: 3, wherein said mutant comprises the four-fold mutation K68S,E114V,N262L,V291C, and wherein said mutant has a sequence identity of at least 80 % to SEQ ID NO: 3.

**7.** The method of one of the preceding claims wherein

a) said AmDH is a mutant of a phenyl alanine dehydrogenase and comprises an amino acid sequence of SEQ ID NO: 2, or of amino acid residues 21 to 400 of SEQ ID NO:2 and/or
b) said AmDH is a mutant of a leucine alanine dehydrogenase comprises an amino acid sequence of SEQ ID NO: 4,and/ or
c) said ADH is selected from AA-ADH comprising an amino acid sequence of SEQ ID NO:5 or an amino acid sequence having a sequence identity of at least 80 % sequence identity, to SEQ ID NO: 5; LBv-ADH comprising an amino acid sequence of SEQ ID NO:6 or an amino acid sequence having a sequence identity of at least 80 % sequence identity, to SEQ ID NO: 6; HL-ADH comprising an amino acid sequence of SEQ ID NO: 8 or an amino acid sequence having a sequence identity of at least 80 % sequence identity, to SEQ ID NO: 8; ADH-A comprising an amino acid sequence of SEQ ID NO: 9 or an amino acid sequence having a sequence identity of at least 80 % sequence identity, to SEQ ID NO: 9; or ADH-hT comprising an amino acid sequence of SEQ ID NO: 10 or an amino acid sequence having a sequence identity of at least 80 % sequence identity, to SEQ ID NO: 10.

**8.** The method of one of the preceding claims wherein said amine is of the general formula I

$$(R_1)(R_2)HC\text{-}NH_2 \qquad (I)$$

wherein
$R_1$ and $R_2$ are identical or different and selected from H, alkyl, cycloalkyl, alkenyl, aryl, aralkyl, alkoxyalkyl, or aryloxyalkyl provided that $R_1$ and $R_2$ are not simultaneously H, and wherein aryl moiety optionally may be substituted.

**9.** The method of one of the preceding claims, which is performed in an aqueous or aqueous-organic mixture reaction medium.

**10.** The method of one of the preceding claims wherein the method is performed in the presence of the set of required enzymes in isolated form, one or more recombinant microorganisms expressing at least one enzyme of the required set of enzymes or cell homogenates or cell lysates of one ore more microorganisms expressing the required set of enzymes or at least one of the enzyme of the required set of enzymes.

**11.** The method of one of the preceding claims wherein the alcohol substrates are selected from monovalent alcohols of the formula II:

$$(R_1)(R_2)HC\text{-}OH \qquad (II)$$

wherein
$R_1$ and $R_2$ are as defined above.

**12.** The method of one of the preceding claims further comprising isolating the produced amine from the reaction medium.

**13.** The method of one of the preceding claims, wherein the cofactor is employed in sub-stoichiometric (catalytic) amounts.

**14.** The method of one of the preceding claims, wherein the reaction is performed in the presence of a (molar) excess of ammonia or of said ammonia source.

**15.** The method of one of the preceding claims wherein the reaction id performed at a pH in the range of lower than 9.6, in particular at about 8.0 to 8.7.

**16.** The method of one of the preceding claims wherein the pH is controlled by means of a buffer which does not show metal chelating properties.

17. The method of one of the preceding claims wherein the employed enzymes are NAD(H) dependent.

18. A recombinant expression vector carrying under the control of at least one regulatory element the coding sequence of at least one ADH and/or at least one AmDH as defined in anyone of the claims 1 to 7.

19. A recombinant microorganism carrying at least one expression vector according to claim 18 and functionally expressing said encoded enzymes.

20. The use of a recombinant expression vector of claim 18 or of a recombinant microorganism of claim 19 in a method as claimed in anyone of the claims 1 to 17.

21. A bioreactor for performing a biocatalytic method of anyone of the claims 1 to 17 containing in a reaction medium at least one recombinant microorganism of claim 19, or at least one enzyme as defined in anyone of the claims 1 to 7 in free or immobilized form.

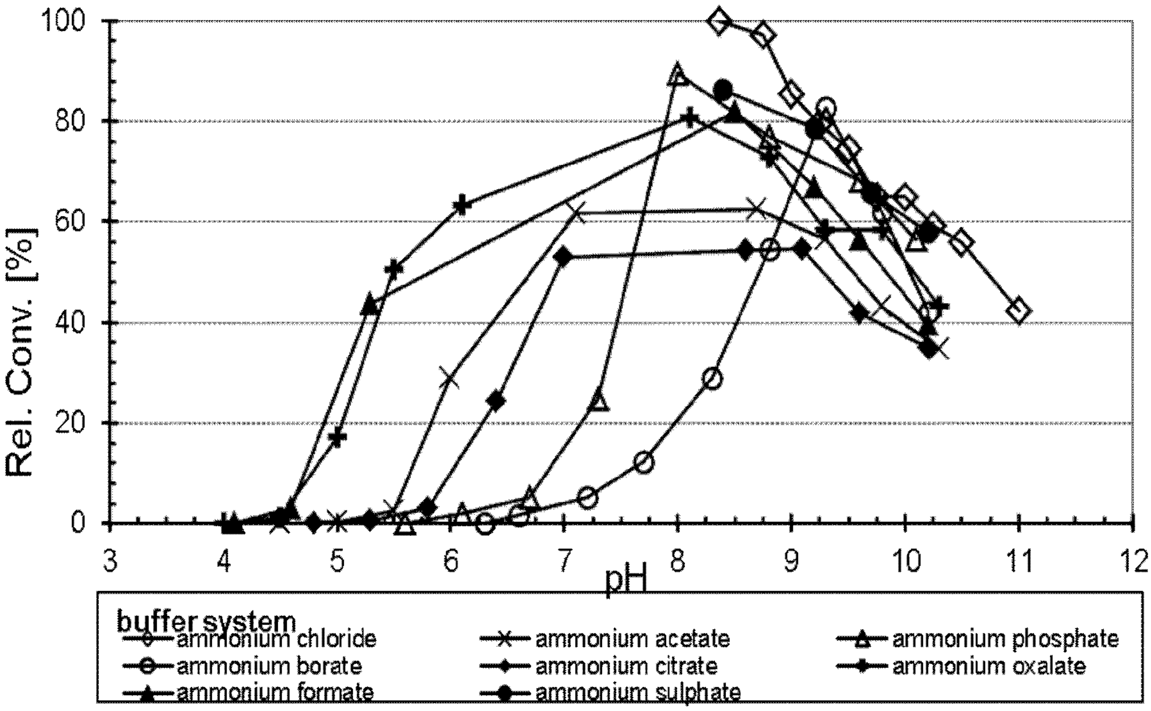
100
80
60
40
20
0
Rel. Conv. [%]
3
4
5
6
7
8
9
10
11
12
pH
buffer system
ammonium chloride
ammonium acetate
ammonium phosphate
ammonium borate
ammonium citrate
ammonium oxalate
ammonium formate
ammonium sulphate

Fig. 1

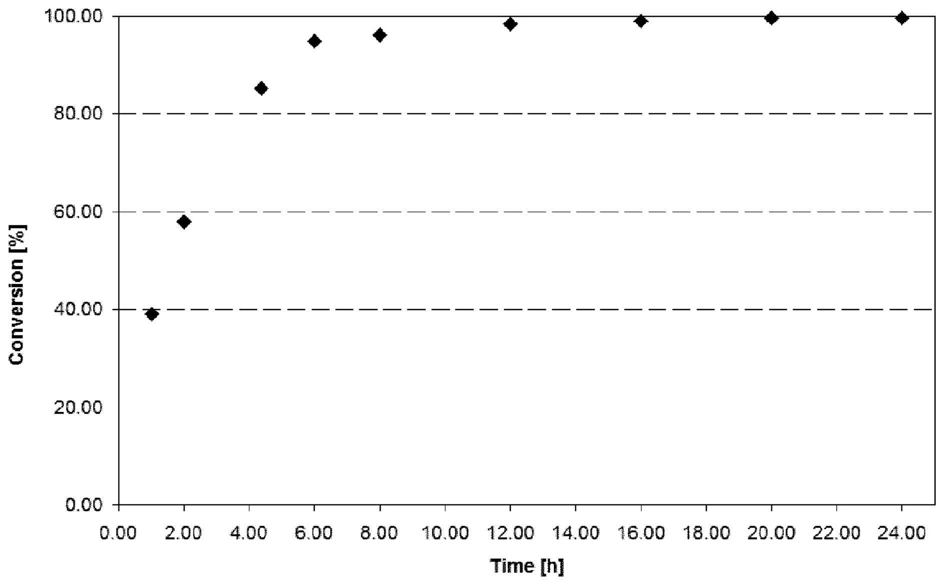
100.00
80.00
60.00
40.00
20.00
0.00
Conversion [%]
0.00
2.00
4.00
6.00
8.00
10.00
12.00
14.00
16.00
18.00
20.00
22.00
24.00
Time [h]

Fig. 2

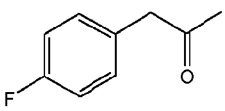
1

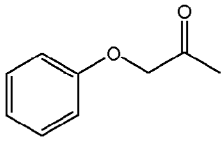
2

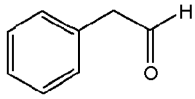
3

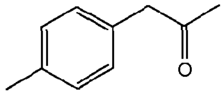
4

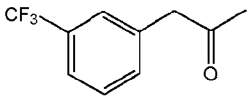
5

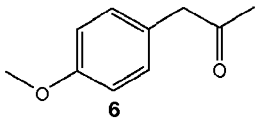
6

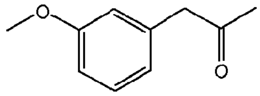
7

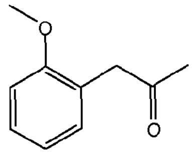
8

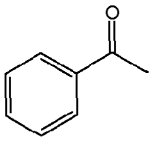
9

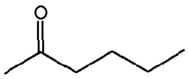
10

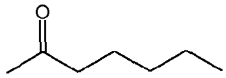
11

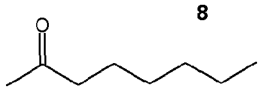
12

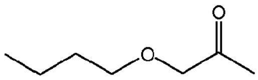
13

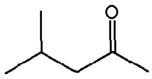
14

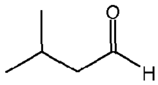
15

Fig. 3

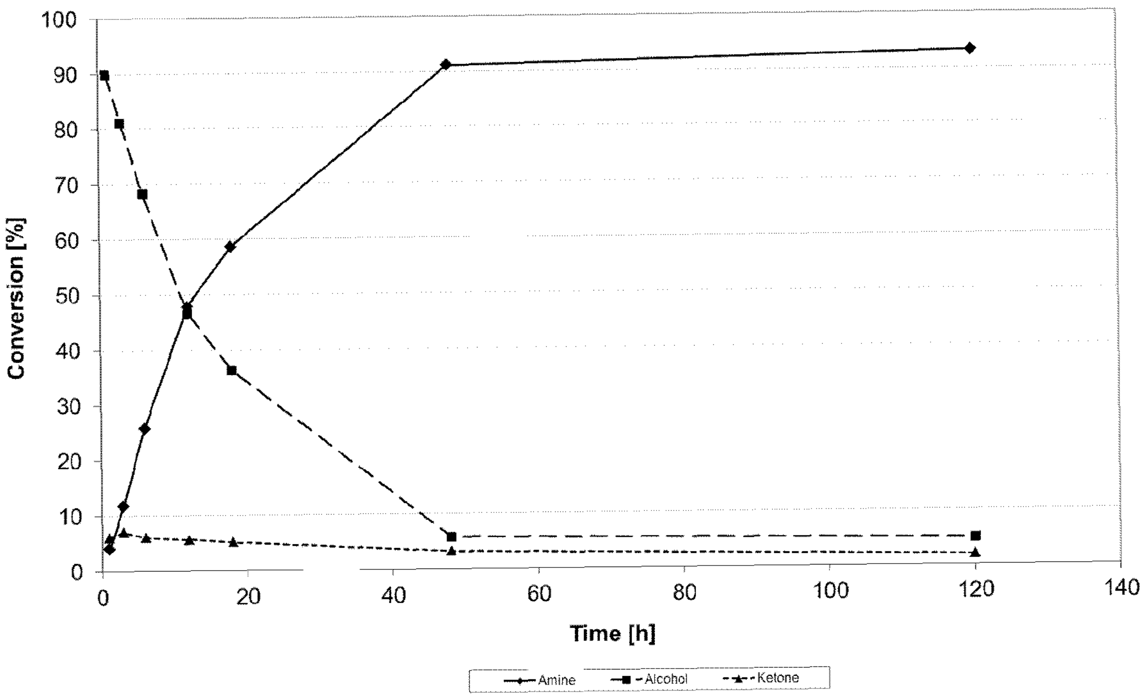


Fig. 4

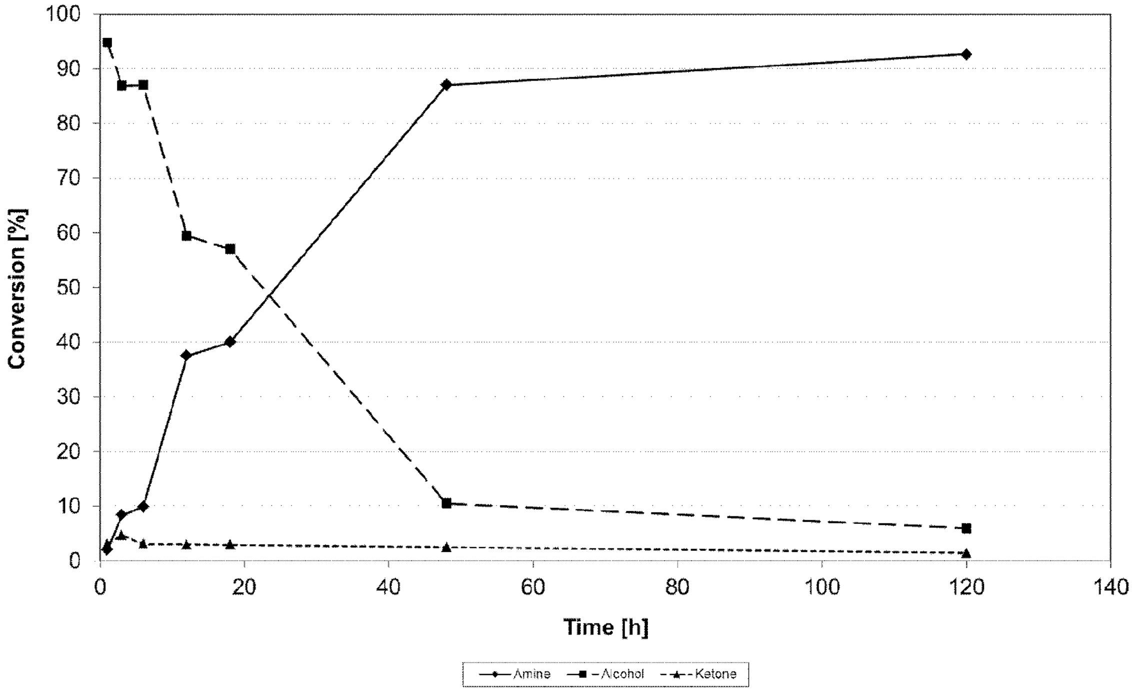
100
90
80
70
60
50
40
30
20
10
0
Conversion [%]
0
20
40
60
80
100
120
140
Time [h]
Amine
Alcohol
Ketone

Fig. 5

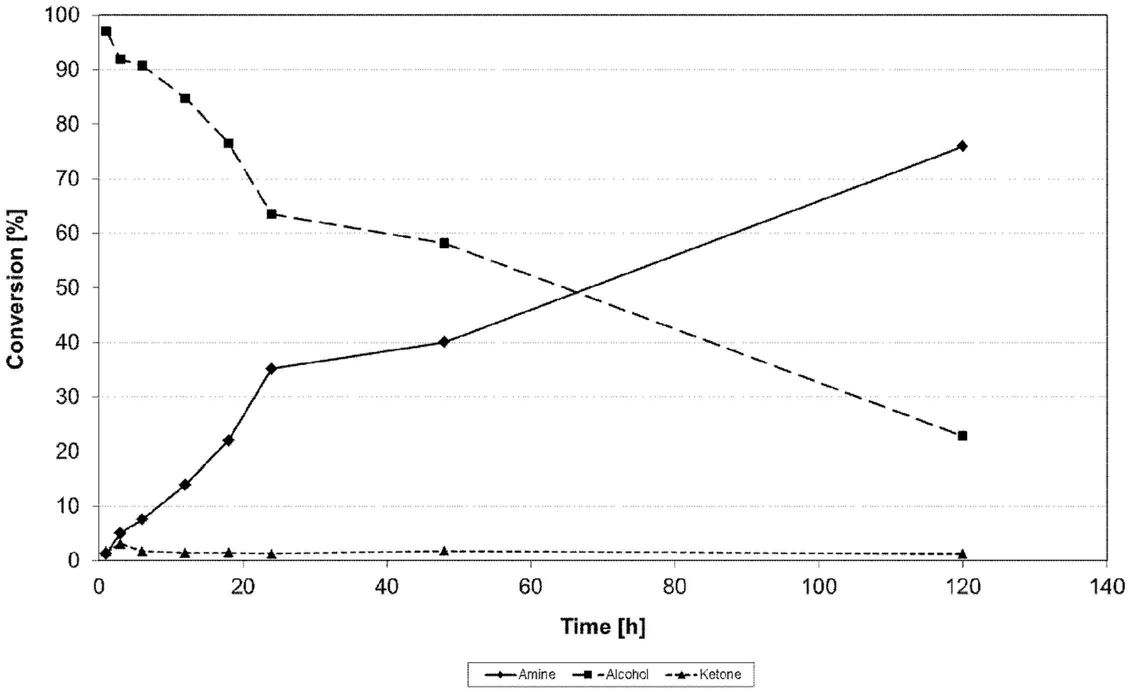
100
90
80
70
60
50
40
30
20
10
0
Conversion [%]
0
20
40
60
80
100
120
140
Time [h]
Amine
Alcohol
Ketone

Fig. 6

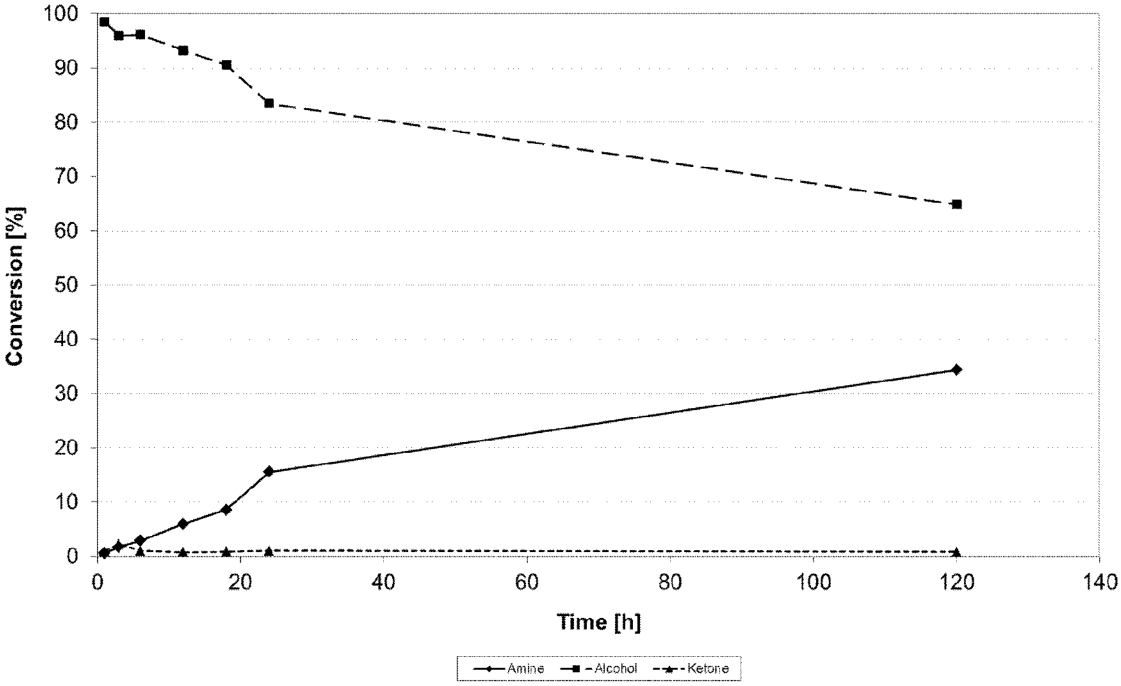
100
90
80
70
60
50
40
30
20
10
0
Conversion [%]
0
20
40
60
80
100
120
140
Time [h]
Amine
Alcohol
Ketone

Fig. 7

| | |
|---|---|
| BAA08816 (1) | --------------------MSLVEKTSIIKDFTLFEKMSEHEQVVFCNDPATGLRAIIAIHDTTLGPALG |
| Translation of B.badius Phe-DH (F. Mutti) (1) | MGSSHHHHHHSSGLVPRGSHMSLVEKTSIIKDFTLFEKMSEHEQVVFCNDPATGLRAIIAIHDTTLGPALG |
| Consensus (1) | --------------------MSLVEKTSIIKDFTLFEKMSEHEQVVFCNDPATGLRAIIAIHDTTLGPALG |
| | |
| BAA08816 (52) | GCRMQPYNSVEEALEDALRLSKGMTYKCAASDVDFGGGKAVIIGDPQKDKSPELFRAFGQFVDSLGGRFYT |
| Translation of B.badius Phe-DH (F. Mutti) (72) | GCRMQPYNSVEEALEDALRLSKGMTYSCAASDVDFGGGKAVIIGDPQKDKSPELFRAFGQFVDSLGGRFYT |
| Consensus (72) | GCRMQPYNSVEEALEDALRLSKGMTY CAASDVDFGGGKAVIIGDPQKDKSPELFRAFGQFVDSLGGRFYT |
| | |
| BAA08816 (123) | GTDMGTNMEDFIHAMKETNCIVGVPEAYGGGGDSSIPTAMGVLYGIKATNKMLFGKDDLGGVTYAIQGLGK |
| Translation of B.badius Phe-DH (F. Mutti) (143) | GTDMGTNMEDFIHAMKETNCIVGVPEAYGGGGDSSIPTAMGVLYGIKATNKMLFGKDDLGGVTYAIQGLGK |
| Consensus (143) | GTDMGTNMEDFIHAMKETNCIVGVPEAYGGGGDSSIPTAMGVLYGIKATNKMLFGKDDLGGVTYAIQGLGK |
| | |
| BAA08816 (194) | VGYKVAEGLLEEGAHLFVTDINEQTLEAIQEKAKTTSGSVTVVASDEIYSQEADVFVPCAFGGVVNDETMK |
| Translation of B.badius Phe-DH (F. Mutti) (214) | VGYKVAEGLLEEGAHLFVTDINEQTLEAIQEKAKTTSGSVTVVASDEIYSQEADVFVPCAFGGVVNDETMK |
| Consensus (214) | VGYKVAEGLLEEGAHLFVTDINEQTLEAIQEKAKTTSGSVTVVASDEIYSQEADVFVPCAFGGVVNDETMK |
| | |
| BAA08816 (265) | QFKVKAIAGSANNQLLTEDHGRHLADKGILYAPDYIVNSGGLIQVADELYEVNKERVLAKTKHIYDAILEV |
| Translation of B.badius Phe-DH (F. Mutti) (285) | QFKVKAIAGSANLQLLTEDHGRHLADKGILYAPDYIVNSGGLIQVADELYEVNKERVLAKTKHIYDAILEV |
| Consensus (285) | QFKVKAIAGSAN QLLTEDHGRHLADKGILYAPDYIVNSGGLIQVADELYEVNKERVLAKTKHIYDAILEV |
| | |
| BAA08816 (336) | YQQAELDQITTMEAANRMCEQRMAARGRRNSFFTSSVKPKWDIRN |
| Translation of B.badius Phe-DH (F. Mutti)(356) | YQQAELDQITTMEAANRMCEQRMAARGRRNSFFTSSVKPKWDIRN |
| Consensus (356) | YQQAELDQITTMEAANRMCEQRMAARGRRNSFFTSSVKPKWDIRN |

**Fig. 8**

```
LeuDH_wt_iso2 (1)     MELFKYMETYDYEQVLFCQDKESGLKAIIAIHDTTLGPALGGTRMWMYNSEEEALEDALRLARGMTYKNAAAGLNLGGGKTVIIG
LeuDH_mut_iso2 (1)    MELFKYMETYDYEQVLFCQDKESGLKAIIAIHDTTLGPALGGTRMWMYNSEEEALEDALRLARGMTYSNAAAGLNLGGGKTVIIG
Consensus (1)         MELFKYMETYDYEQVLFCQDKESGLKAIIAIHDTTLGPALGGTRMWMYNSEEEALEDALRLARGMTY NAAAGLNLGGGKTVIIG

LeuDH_wt_iso2 (86)    DPRKDKNEAMFRAFGRFIQGLNGRYITAEDVGTTVADMDIIYQETDYVTGISPEFGSSGNPSPATAYGVYRGMKAAAKEAFGSDS
LeuDH_mut_iso2 (86)   DPRKDKNEAMFRAFGRFIQGLNGRYITAVDVGTTVADMDIIYQETDYVTGISPEFGSSGNPSPATAYGVYRGMKAAAKEAFGSDS
Consensus (86)        DPRKDKNEAMFRAFGRFIQGLNGRYITA DVGTTVADMDIIYQETDYVTGISPEFGSSGNPSPATAYGVYRGMKAAAKEAFGSDS

LeuDH_wt_iso2 (171)   LEGKVVAVQGVGNVAYHLCRHLHEEGAKLIVTDINKEVVARAVEEFGAKAVDPNDIYGVECDIFAPCALGGIINDQTIPQLKAKV
LeuDH_mut_iso2 (171)  LEGKVVAVQGVGNVAYHLCRHLHEEGAKLIVTDINKEVVARAVEEFGAKAVDPNDIYGVECDIFAPCALGGIINDQTIPQLKAKV
Consensus (171)       LEGKVVAVQGVGNVAYHLCRHLHEEGAKLIVTDINKEVVARAVEEFGAKAVDPNDIYGVECDIFAPCALGGIINDQTIPQLKAKV

LeuDH_wt_iso2 (256)   IAGSADNQLKEPRHGDIIHEMGIVYAPDYVINAGGVINVADELYGYNRERAMKKIEQIYDNIEKVFAIAKRDNIPTYVAADRMAE
LeuDH_mut_iso2 (256)  IAGSADLQLKEPRHGDIIHEMGIVYAPDYVINAGGCINVADELYGYNRERAMKKIEQIYDNIEKVFAIAKRDNIPTYVAADRMAE
Consensus (256)       IAGSAD QLKEPRHGDIIHEMGIVYAPDYVINAGG INVADELYGYNRERAMKKIEQIYDNIEKVFAIAKRDNIPTYVAADRMAE

LeuDH_wt_iso2 (341)   ERIETMRKAASQFLQNGHHILSRRPRPLTAARAGLRRADDGGTTTMQEQKFRILTINPGSTSTKIGVFENERAIASKKRSATRAG
LeuDH_mut_iso2 (341)  ERIETMRKAASQFLQNGHHILSRRPRPLTAARAGLRRADDGGTTTMQEQKFRILTINPGSTSTKIGVFENERAIASKKRSATRAG
Consensus (341)       ERIETMRKAASQFLQNGHHILSRRPRPLTAARAGLRRADDGGTTTMQEQKFRILTINPGSTSTKIGVFENERAIASKKRSATRAG

LeuDH_wt_iso2 (426)   ASAI
LeuDH_mut_iso2 (426)  ASAI
Consensus (426)       ASAI
```

**Fig. 9**

Europäisches Patentamt
European Patent Office
Office européen des brevets

# EUROPEAN SEARCH REPORT

Application Number
EP 14 17 5632

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/309734 A1 (BOMMARIUS ANDREAS SEBASTAIN [US] ET AL) 21 November 2013 (2013-11-21) * abstract; figures 1, 2, 13; examples 1-4 * * paragraph [0230] * ----- | 1-17 | INV. C12P13/00 C12N9/06 |
| X,D | MICHAEL J. ABRAHAMSON ET AL: "The Evolution of an Amine Dehydrogenase Biocatalyst for the Asymmetric Production of Chiral Amines", ADVANCED SYNTHESIS & CATALYSIS, vol. 355, no. 9, 17 June 2013 (2013-06-17), pages 1780-1786, XP055157345, ISSN: 1615-4150, DOI: 10.1002/adsc.201201030 * abstract * * scheme 1; page 1781 * * page 1782, right-hand column, paragraph 2 - page 1783, left-hand column, paragraph 2; tables 2, 3 * ----- | 1-17 | TECHNICAL FIELDS SEARCHED (IPC) C12N C12P |
| X,D | MICHAEL J. ABRAHAMSON ET AL: "Development of an Amine Dehydrogenase for Synthesis of Chiral Amines", ANGEWANDTE CHEMIE INTERNATIONAL EDITION, vol. 51, no. 16, 6 March 2012 (2012-03-06), pages 3969-3972, XP055157347, ISSN: 1433-7851, DOI: 10.1002/anie.201107813 * schemes 1 and 2; page 3969, right-hand column, paragraph 2 - page 3972, left-hand column, paragraph 1; tables 2, 3 * ----- -/-- | 1-17 | |

1

~~The present search report has been drawn up for all claims~~

| Place of search | Date of completion of the search | Examiner |
|---|---|---|
| Berlin | 10 December 2014 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 17 5632

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VERENA RESCH ET AL: "Deracemisation of Mandelic Acid to Optically Pure Non-Natural L-Phenylglycine via a Redox-Neutral Biocatalytic Cascade", ADVANCED SYNTHESIS & CATALYSIS, vol. 352, no. 6, 19 April 2010 (2010-04-19), pages 993-997, XP055015033, ISSN: 1615-4150, DOI: 10.1002/adsc.200900891 * abstract * * scheme 1; page 993 * ----- | 1-4 | |
| A | SELIN KARA ET AL: "Recent trends and novel concepts in cofactor-dependent biotransformations", APPLIED MICROBIOLOGY AND BIOTECHNOLOGY, vol. 98, no. 4, 21 December 2013 (2013-12-21), pages 1517-1529, XP055157481, ISSN: 0175-7598, DOI: 10.1007/s00253-013-5441-5 * scheme 9; page 1523, left-hand column, paragraph 1 * * scheme 13; page 1524, right-hand column, paragraph 2 * ----- | 1,2 | TECHNICAL FIELDS SEARCHED (IPC) |
| X | EP 1 020 515 A2 (DAICEL CHEM [JP]) 19 July 2000 (2000-07-19) * abstract; claim 17; examples 9, 10; table 8 * ----- | 1,9,10, 12,14-17 | |

-/--

~~The present search report has been drawn up for all claims~~

1

| Place of search | Date of completion of the search | Examiner |
|---|---|---|
| Berlin | 10 December 2014 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 17 5632

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | HANNES KOHLS ET AL: "Recent achievements in developing the biocatalytic toolbox for chiral amine synthesis", CURRENT OPINION IN CHEMICAL BIOLOGY, vol. 19, 1 April 2014 (2014-04-01), pages 180-192, XP055157361, ISSN: 1367-5931, DOI: 10.1016/j.cbpa.2014.02.021 * page 182, right-hand column, paragraph 2-4 * | 1-17 | |
| A | WO 02/36742 A2 (BIOCATALYTICS INC [US]; ROZZELL J DAVID JR [US]) 10 May 2002 (2002-05-10) * abstract; claims 18-21; examples 26, 29 * * page 8, line 3 - page 9, line 25 * | 1-17 | |
| A | US 2013/029385 A1 (CABIROL FABIEN L [DE] ET AL) 31 January 2013 (2013-01-31) * abstract * * paragraphs [0136], [0137] * | 1-7 | TECHNICAL FIELDS SEARCHED (IPC) |

1 ~~The present search report has been drawn up for all claims~~

| Place of search | Date of completion of the search | Examiner |
|---|---|---|
| Berlin | 10 December 2014 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 14 17 5632

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-17

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 14 17 5632

The Search Division considers that the present European patent application does not comply with the requirements of unity of invention and relates to several inventions or groups of inventions, namely:

1. claims: 1-17

   Method for preparing an amine

---

2. claims: 18-20

   Recombinant expression vector and its uses

---

3. claim: 21

   Bioreactor

---

## ANNEX TO THE EUROPEAN SEARCH REPORT ON EUROPEAN PATENT APPLICATION NO.

EP 14 17 5632

This annex lists the patent family members relating to the patent documents cited in the above-mentioned European search report.
The members are as contained in the European Patent Office EDP file on
The European Patent Office is in no way liable for these particulars which are merely given for the purpose of information.

10-12-2014

| Patent document cited in search report | Publication date | Patent family member(s) | Publication date |
|---|---|---|---|
| US 2013309734 A1 | 21-11-2013 | NONE | |
| EP 1020515 A2 | 19-07-2000 | EP 1020515 A2 | 19-07-2000 |
| | | US 6432688 B1 | 13-08-2002 |
| | | US 2003027306 A1 | 06-02-2003 |
| WO 0236742 A2 | 10-05-2002 | AU 3260302 A | 15-05-2002 |
| | | US 2002061564 A1 | 23-05-2002 |
| | | US 2008076162 A1 | 27-03-2008 |
| | | WO 0236742 A2 | 10-05-2002 |
| US 2013029385 A1 | 31-01-2013 | US 2013029385 A1 | 31-01-2013 |
| | | WO 2011100265 A2 | 18-08-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1149849 A
- EP 1069183 A
- DE OS100193773
- WO 2005108590 A
- DE 19610984 A1
- US 7569375 B


### Non-patent literature cited in the description


- **K. WEISSERMEL ; H.-J. ARPE.** Industrial Organic Chemistry. Wiley-VCH, 2003, 51
- **S. IMM ; S. BÄHN ; M. ZHANG ; L. NEUBERT ; H. NEUMANN ; F. KLASOVSKY ; J. PFEFFER ; T. HAAS ; M. BELLER.** *Angew. Chem. Int. Ed.,* 2011, vol. 50, 7599-7603
- **Y. ZHANG ; C.-S. LIM ; D. S. B. SIM ; H.-J. PAN ; Y. ZHAO.** *Angew. Chem. Int. Ed.,* 2014, vol. 126, 1423-1427
- **J. H. SATTLER ; M. FUCHS ; K.TAUBER ; F. G. MUTTI ; K. FABER ; J. PFEFFER ; T. HAAS ; W. KROUTIL.** *Angew. Chem. Int. Ed.,* 2012, vol. 51, 9156-9159
- **J.-S. SHIN ; B.-G. KIM.** *Biosci. Biotechnol. Biochem.,* 2001, vol. 65, 1782-1788
- **J.-S. SHIN ; B.-G. KIM.** *Biotechnol. Bioeng.,* 1999, vol. 65, 206-211
- **J.-S. SHIN ; B.-G. KIM.** *Biotechnol. Bioeng.,* 2002, vol. 77, 832-837
- **ABRAHAMSON, M.J. et al.** *Angew. Chem. Int Ed,* 2012, vol. 51, 3969
- **PEARSON ; LIPMAN.** *Proc. Natl. Acad, Sci. (USA),* 1988, vol. 85 (8), 2444-2448
- **NARANG, S.A.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- **HIGGINS DG ; SHARP PM.** Fast and sensitive multiple sequence alignments on a microcomputer. *Comput Appl. Biosci.,* April 1989, vol. 5 (2), 151-1
- **CHENNA, RAMU ; SUGAWARA, HIDEAKI ; KOIKE,TADASHI ; LOPEZ, RODRIGO ; GIBSON, TOBY J ; HIGGINS, DESMOND G ; THOMPSON, JULIE D.** Multiple sequence alignment with the Clustal series of programs. *Nucleic Acids Res,* 2003, vol. 31 (13), 3497-500, http://www.ebi.ac.uk/Tools/clustalw/index.html#
- Voet, Voet. Wiley Press, 896-897
- **SAMBROOK, J. ; FRITSCH, E.F. ; MANIATIS, T.** Molecular Cloning: A Laboratory Manual. Cold Spring Harbor Laboratory Press, 1989
- Current Protocols in Molecular Biology. John Wiley & Sons, 1985
- Nucleic Acids Hybridization: A Practical Approach. IRL Press at Oxford University Press, 1985
- Essential Molecular Biology: A Practical Approach. IRL Press at Oxford University Press, 1991
- **SAMBROOK, J. ; FRITSCH, E.F. ; MANIATIS, T.** Molecular Cloning (A Laboratory Manual). Cold Spring Harbor Laboratory Press, 1989, 9.31-9.57
- Current Protocols in Molecular Biology. John Wiley & Sons, 1989, 6.3.1-6.3.6
- **SAMBROOK ; RUSSELL.** Molecular Cloning. Cold Spring Harbor Laboratory Press, 2001
- In vitro mutagenesis protocols. Humana Press, 1996
- **KEGLER-EBO DM ; DOCKTOR CM ; DIMAIO D.** *Nucleic Acids Res,* 1994, vol. 22, 1593
- **BARETTINO D ; FEIGENBUTZ M ; VALCAREL R ; STUNNENBERG HG.** *Nucleic Acids Res,* 1994, vol. 22, 541
- **BARIK S.** *Mol Biotechnol,* 1995, vol. 3, 1
- **ECKERT KA ; KUNKEL TA.** *Nucleic Acids Res,* 1990, vol. 18, 3739
- **SCHENK et al.** *Biospektrum,* 2006, vol. 3, 277-279
- An efficient random mutagenesis technique using an E.coli mutator strain. **GREENER A ; CALLAHAN M ; JERPSETH B.** In vitro mutagenesis protocols. Humana Press, 1996
- **STEMMER WPC.** *Nature,* 1994, vol. 370, 389

- **STEMMER WPC.** *Proc Natl Acad Sci USA,* 1994, vol. 91, 10747
- **REETZ MT ; JAEGER K-E.** *Topics Curr Chem,* 1999, vol. 200, 31
- Methods for optimizing industrial enzymes by directed evolution. **ZHAO H ; MOORE JC ; VOLKOV AA ; ARNOLD FH.** Manual of industrial microbiology and biotechnology. American Society for Microbiology, 1999
- **GOEDDEL.** Gene Expression Technology: Methods in Enzymology. Academic Press, 1990, vol. 185
- book Cloning Vectors. Elsevier, 1985
- **T. MANIATIS ; E.F. FRITSCH ; J. SAMBROOK.** Molecular Cloning: A Laboratory Manual. Cold Spring Harbor Laboratory, 1989
- **T.J. SILHAVY ; M.L. BERMAN ; L.W. ENQUIST.** Experiments with Gene Fusions. Cold Spring Harbor Laboratory, 1984
- **AUSUBEL, F.M. et al.** Current Protocols in Molecular Biology. Greene Publishing Assoc. and Wiley Interscience, 1987
- **POUWELS P.H. et al.** Cloning Vectors. Publ. Elsevier, 1985
- Current Protocols in Molecular Biology. Publ. Wiley Interscience, 1997
- **SAMBROOK et al.** Molecular Cloning: A Laboratory Manual. Cold Spring Harbor Laboratory Press, 1989
- **CHMIEL.** Bioprozesstechnik 1. Einführung in die Bioverfahrenstechnik. Gustav Fischer Verlag, 1991
- **STORHAS.** Bioreaktoren und periphere Einrichtungen. Vieweg Verlag, 1994
- Manual of Methods for General Bacteriology. American Society for Bacteriology, 1981
- **P.M. RHODES ; P.F. STANBURY.** Applied Microbiol. Physiology, A Practical Approach. IRL Press, 1997, 53-73
- Immobilization of Enzymes. **J. LALONDE ; A. MARGOLIN.** Enzyme Catalysis in Organic Synthesis. Wiley-VCH, 2002, vol. III, 991-1032
- **S. NAGATA ; K. TANIZAWA ; N. ESAKI ; Y. SAKAMOTO ; T. OHSHIMA ; H. TANAKA ; K. SODA.** *Biochemistry,* 1988, vol. 27, 9056-9062
- **A. YAMADA ; T. DAIRI ; Y. OHNO ; X.-L. HUANG ; Y. ASANO.** *Biosci. Biotech. Biochem.,* 1995, vol. 59 (10), 1994-1995
- **LAMPEL, K.A. et al.** *Journal of Bacteriology,* 1986, vol. 166 (1), 238
- **ABRAHAMSON, M.J. et al.** *Advanced Synthesis and Catalysis,* 2013, vol. 355 (9), 1780-1786
- **ASANO, Y. et al.** *European Journal of Biochemistry,* 1987, vol. 188 (1), 153
- **CARLSON, R.** *Nat Biotechnol.,* 2009, vol. 27, 1091
- **BASF SE ; HÖFFKEN, H.W. et al.** *Biochemistry,* 2006, vol. 45 (1), 82
- **SCHLIEBEN, N.H. et al.** *Journal of Molecular Biology,* 2005, vol. 349 (4), 801
- **PARK, H.D. et al.** *J. boil. Chem.,* 1991, vol. 266 (20), 13296
- **KOSJEK et al.** *Biotechnol. Bioeng.,* 2004, vol. 86 (1), 55
- **R. CANNIO ; M. ROSSI ; S. BARTOLUCCI.** *Eur. J. Biochem.,* 1994, vol. 222, 345-352
- **X. ZHANG ; T. C. BRUICE.** *Biochemistry,* 2007, vol. 46, 837-843
- **SAMBROOK J ; RUSSELL DW.** Molecular Cloning: a Laboratory Manual. Cold Spring. Harbor Laboratory Press, 2001
- **D. H. PARK ; B. V. PLAPP.** *J. Biol. Chem.,* 1991, vol. 266, 13296-13302
- **S. AL-KARADAGHI ; E. S. CEDERGREN-ZEPPEZAUER ; S. HOVMOLLER ; K. PETRATOS ; H. TERRY ; K. S. WILSON.** *Acta Crystallogr. Sect. D,* 1994, vol. 50, 793-807
- **V. PRELOG.** *Pure Appl. Chem.,* 1964, vol. 9, 119-130
- **B. KOSJEK ; W. STAMPFER ; M. POGOREVC ; W. GOESSLER ; K. FABER ; W. KROUTIL.** *Biotechnol. Bioeng.,* 2004, vol. 86, 56-62
- **K. INOUE ; Y. MAKINO ; T. DAIRI ; N ITOH.** *Biosci. Biotechnol. Biochem.,* 2006, vol. 70, 418-426
- **S. LEUCHS ; L. GREINER.** *Chem.Biochem.Eng.Q.,* 2011, vol. 25, 267-281
- **N. H. SCHLIEBEN ; K. NIEFIND ; J. MULLER ; B. RIEBEL ; W. HUMMEL ; D. SCHOMBURG.** *J.Mol.Biol.,* 2005, vol. 349, 801-813
- **H. W. HÖFFKEN ; DUONG MINH ; T. FRIEDRICH ; M. BREUER ; B. HAUER ; R. REINHARDT ; R. RABUS ; J. HEIDER.** *Biochemistry,* 2006, vol. 45, 82-93
- **CARLSON, R.** *Nat. Biotechnol.,* 2009, vol. 27, 1091